(19)

Europäisches
Patentamt
European
Patent Office
Office européen
des brevets

(11) **EP 1 968 898 B1**

(12) **EUROPEAN PATENT SPECIFICATION**

(45) Date of publication and mention
of the grant of the patent:
**24.07.2013 Bulletin 2013/30**

(51) Int Cl.:
*C02F 1/34* (2006.01)     *C02F 1/28* (2006.01)
*C02F 101/32* (2006.01)

(21) Application number: **06808470.6**

(86) International application number:
**PCT/GB2006/004176**

(22) Date of filing: **08.11.2006**

(87) International publication number:
**WO 2007/054691 (18.05.2007 Gazette 2007/20)**

(54) **BIOREMEDIATION MATERIALS**

MATERIALIEN FÜR DIE BIOLOGISCHE SCHADSTOFFENTFRACHTUNG

MATERIAUX DE BIOREMEDIATION

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **08.11.2005 GB 0522773**

(43) Date of publication of application:
**17.09.2008 Bulletin 2008/38**

(73) Proprietors:
• **The University of Surrey
Guildford Surrey GU2 7XH (GB)**
• **The Forestry Commission
Edinburgh
EH12 7AT (GB)**

(72) Inventors:
• HUTCHINGS, Tony, Richard
Surrey GU10 4LH (GB)
• DE LEIJ, Franciscus, Antonius, Anna, Maria
West Sussex BN16 3NG (GB)
• WINGATE, Jeremy, Robert
Essex CM9 8EE (GB)

(74) Representative: **Hutter, Anton
Venner Shipley LLP
200 Aldersgate
London EC1A 7HD (GB)**

(56) References cited:
EP-A2- 0 646 642     EP-A2- 1 178 017
DE-A1- 19 928 087     JP-A- 2005 021 748
US-A- 6 008 028     US-A1- 2004 031 751
US-B1- 6 451 580

## Description

FIELD OF THE INVENTION

The present invention relates to charcoal for use in bioremediation, methods for its manufacture and the selection of materials that allow enhanced survival of the microbes that are associated with the charcoal.

INTRODUCTION

Pollution of land, water and air with organic contaminants, such as hydrocarbons, and including polycyclic aromatic hydrocarbons (PAHs), BTEX (benzene, toluene, ethylbenzene and xylenes) hydrocarbons, TCE (trichloroethylene) and (chlorinated) phenols is a major problem. These pollutants arise from industrial processes and can affect soil quality, or partially dissolve in groundwater where they are difficult to retain, causing potential harm to surface and ground waters, humans, and adjacent ecological receptors.

An example of a procedure for dealing with contaminated groundwaters is pump-and-treat, in which water is pumped from a contaminated site and treated. Some pollutants, such as TCE, that have a greater density than water, are difficult to remove using pump and treat, especially where solubility in water is low. This means that vast quantities of water have to be used to remove small quantities of pollutant. The pump-and-treat process is therefore very slow and costly and, depending on the pollutant, can require at least 10 years in order to reduce dissolved contaminants to below clean water standards (Voudrias EA, Global Nest: the Int. J. Vol 3, No. 1, pp 1-10, 2001).

Additionally, surface treatment of the pumped water typically requires the use of activated (amorphous) carbon to remove pollutants. The charcoal has to be continually replaced, and treated at very high temperatures to destroy the pollutants. Process costs are inevitably high. Methods for dealing with contaminated land itself include thermal desorption of pollutants, soil vapour extraction, and landfill of contaminated soil (dig and dump). All these methods are costly and have a large environmental impact.

*In situ* bioremediation of TCE in groundwater aquifers is known, specifically in the form of a continuous system to replace a pump-and-treat system, the bioremediation system requiring the pumping into a polluted aquifer of toluene such that levels in the aquifer reach about 25 parts per billion, thus causing native bacteria to produce toluene monooxygenase (which also catalyses the degradation of TCE), and the continuous pumping of hydrogen peroxide in order to supply oxygen to the native bacteria. However, the efficacy of this technique is known to be limited for a number of reasons including the limited ability of the bacteria to stay attached to the soil in the aquifer.

Bioaugmentation is also known in which a similar method is used, additionally augmenting the native population of bacteria with a bacterium or bacteria which are known to be able to break down the pollutant. For example, a chosen bacterium can be used in bioaugmentation to treat TCE, the chosen bacterium not producing the chloroform which is typically produced by bacteria when they degrade TCE.

Heavily polluted sites may simply be left undeveloped for prolonged periods whilst the pollutant levels decrease to acceptable levels. This can take decades and during that time the land is not suitable for habitation, farming, or industrial use and this can result in the land becoming an eyesore, and means that potentially valuable land is left unused at a time when it would preferably be put to constructive commercial or beneficial use.

In the treatment of waste water, biological methods are often employed where porous clean materials with large surface areas are used to support microbial biofilms that are colonised by a community of microbes. To encourage the development of a microbial community that has a large proportion of organisms that are capable of degrading the pollutants that are present in the waste stream in the first instance, a period of priming is required where waste water is filtered over the support material allowing the biofilm to establish. Inevitably, during this period, biological degradation is less than optimal and it can take several weeks to months before an effective microbial community has established on the support material. In the case of biological water filtration, materials that have been used to support microbial biofilms include, ceramic beads and tubes, volcanic rocks, graded sand (slow sand filters) and granulated activated charcoal (GAC). This method of using a clean porous material and circulating a pollutant containing effluent over this material to encourage the establishment of a microbial community capable of degrading the added pollutants has been adapted for soil systems by Takagi et al., in US-A-6451580, below.

Takagi *et al.,* disclose, in US-A-6451580 a method for the enrichment of a soil layer to which a porous material, such as charcoal is added. A suspension containing minerals and pollutants is then added to the soil and charcoal mixture. The charcoal adsorbs contaminants which are chlorinated pesticides that have a greater affinity for the charcoal than for the soil matrix, allowing the pesticides to become desorbed from the soil matrix and becoming bound to the charcoal where they can subsequently be degraded by microbes that become associated with the charcoal. Enriched soil can be used to repeat the process.

US6451580B discloses a method of enriching bacteria, the method comprising mixing charcoal into a soil which contaminant-decomposing bacteria inhabit to form an enrichment soil layer. An inorganic salt medium containing only a kind of organic contaminant to be decomposed is caused to circulate continuously to the enrichment soil layer enabling enrichment of the contaminant-decomposing bacteria.

There remains a need for cheap and effective means for the treatment of polluted environments, especially those polluted by hydrocarbons, which can be ecologically disastrous.

Surprisingly, we have now discovered that it is possible to colonise charred biological material, such as charcoal, with micro-organisms effective to metabolise a selected environmental substance, by seeding the material with the substance.

SUMMARY OF THE INVENTION

Thus, in a first aspect, the present invention provides claim 1.

Therefore, the colonising microbe, also referred to herein as an organism, is tolerant to the presence to of the pollutant and is capable of metabolising said pollutant. A selective amount of the pollutant, as discussed elsewhere, is for instance that capable of exerting a selection pressure on non-tolerant organisms.

Therefore, the presence of the selected pollutant can be seen as being advantageous for the colonising microbes, thereby allowing preferential colonisation of the charcoal used in the method of the invention by the microbes that are capable of metabolising the substance or are tolerant to it. Thus, the pollutant is present in the charred material in an amount sufficient to provide a positive selection pressure on the microbes that are capable of metabolising the pollutant. The terms colonising microbes or metabolising microbes can be used interchangeably.

The pollutant is selective *for* the colonising/metabolising microbes and, therefore, may be selective *against* other microbes that cannot metabolise said pollutant or lead to the promotion of metabolism of the pollutant. The pollutant, therefore, preferably leads to a negative or disadvantageous selection pressure on other microbes that cannot metabolise the pollutant, such that its presence is non-advantageous to said non-metabolising organisms. The advantage of this is that competition for resources is reduced so that the metabolising microbes of the present invention thrive and, therefore when used in situ, an increase in the total levels of metabolism of the pollutant are seen, leading to a reduction in the levels of the substance in the environment.

The levels of the pollutant in the environment are reduced, or the rate of "de-contamination" of the area is increased, by the use of the present invention.

It will be understood that metabolism of the selected pollutant preferably reduces any detrimental effects that the pollutant may have on the environment. The pollutant is preferably destroyed or chemically altered, or its detrimental activity reduced, as a result of its metabolism (the process of it being metabolised) by the colonising microbes.

Microbes that lead to the promotion of metabolism of the pollutant include other microbes that help to support the metabolising microbes (microbes that are capable of metabolising the substance) or microbes that are capable of metabolising the breakdown products of the substance, for instance, as discussed below.

Thus, it is important to distinguish between the "selected" pollutant, which can be considered as that chosen by the bioremediator, and the "selective" effect of that pollutant, i.e. the exertion of a selection pressure, on the colonising microbes.

Wood charcoal is generally preferred.

References to charcoal include references to other charred biological material, unless otherwise apparent.

The term "environmental" is used herein to indicate a pollutant with which the colonising microbes in the charred material can interact when deployed *in situ.* Such interaction may only occur once further treatment, such as heating or irrigation, has occurred or been effected.

A particular advantage of charcoal used in the method is that it provides an inert, protective matrix for the microbes and that, because of its structure, the micro-organisms are able to populate the charcoal to far higher densities than may be found in soil, with levels of between 100 and 1,000,000 times greater than those found in soil being common. Charcoal colonised in accordance with the present invention often has quantities of > $10^9$ bacteria per gram of charcoal. Depending on the type of charcoal used in the method and the colonisation level within the charcoal, microbes are largely protected against physical and chemical stress. Even when levels drop after a period of dry storage, levels can quickly be restored on hydration and the addition of nutrients where necessary.

The environment provided by charcoal is, effectively, a highly porous network, or matrix, which is readily colonised by micro-organisms to form a bio-film. Such a bio-film will generally be a community of different cooperating microbes. Such a community is generally more effcient and stable in the environment from which it has been selected than any individual microbial strain making up that community, under similar circumstances. It will be appreciated that some strains may actually thrive when grown as a single population, possibly even better than when they were part of a multi-species community.

Other strains, however, may need a commensal where one organism degrades the waste products of another, leading to enhanced survival, while in other cases different species may add to the stability and integrity of the community, for example via the production of extra-cellular polymers that retain water and provide protection against toxins. Other microbes might detoxify their environment via the production of carbonates and sulphides that cause the precipitation of toxic metal ions. It is preferred, in fact, that the charcoal used in the method also comprises further organisms which are capable of interacting with the colonising bacteria, thus raising the metabolism of the pollutant. Thus, it is particularly preferred that combinations of microbes, i.e. more than one distinct species or strain, are provided.

The microbes of the present invention may be any that is suitable, and include bacteria, fungi and protozoa. Bacteria and fungi are more preferred, especially bacteria. It will be understood that reference to bacteria, hereon in, also includes reference to other microbes suitable for use in the present invention, unless the context specifically requires otherwise.

It is important that the bacteria be selected for their ability to metabolise the selected environmental pollutant. In this regard, the pollutant may be any that is metabolisable by a microbe. The pollutant may be any that it is desirable to metabolise, whether it be to increase levels of advantageous chemicals in the environment by releasing bound ingredients, for example, or to catabolise environmental toxins or undesirable waste substances, such as pollutants and odours, respectively. The term 'selected' indicates a pollutant which has been selected in circumstances where the colonised charcoal of the present invention may be used to metabolise it.

It is also preferred that the colonising bacteria or microbes are capable of metabolising or breaking down the further break down products of the pollutant itself, thus preventing a build-up of the metabolite or break-down product, which could affect the equilibrium position or rate of removal of the pollutant *per se.*

Preferably, the selected pollutant is tolerated only by the colonising bacteria or organisms, to the exclusion of other bacteria or organisms that may compete with the colonising bacteria or organisms for essential nutrients. In other words, in order to maximize the metabolism or breakdown of the selected pollutant, it is preferred that the charcoal is predominantly colonised by the bacteria or organisms capable of metabolising or breaking down said pollutant.

The pollutant, may be a hydrocarbon, especially crude oil, petroleum or diesel, or polycyclic, aromatic hydrocarbons. In this instance, it is preferred that the charcoal used in the method is colonised with bacteria, for instance, that not only are capable of tolerating the presence of the hydrocarbon, which would be toxic in even tiny concentrations to a great number of organisms, but the bacteria are also capable of metabolising or breaking down the pollutant, thus reducing its concentration *in situ.* In order to establish suitable conditions for bacteria that are capable of metabolising the pollutant, the pollutant is actually added to the charcoal. The advantage of this is that bacteria and other organisms that may compete with the colonising bacteria (those that are capable of metabolising the pollutant) for other nutrients, such a minerals etc, are reduced in number and out-competed by those organisms that can utilise the said pollutants such that the selection pressure exerted by adding a selective substance into the charcoal makes the microbial populations of interest (those that can degrade a specific pollutant for example) thrive, leading to greater reductions in the pollutant levels, over time.

In fact, none of the prior art teaches towards providing the substance within the charred material so as to select for organisms that can metabolise the pollutant. In the above example, this translates as adding the pollutant to the charcoal to exert such a selection pressure.

A preferred example of a suitable mineral solution is Basal Salt Medium (BSM) comprising Vitamins and trace elements as shown in Table 1 in the Examples.

<u>BRIEF DESCRIPTION OF THE FIGURES</u>

The Figures will be described, wherein:

Figure 1. Bacterial colonisation of I gram charcoal particles with various particle sizes; (Fine: <0.5 mm; Small: 0.5 - 2 mm; Medium: 2-5 mm; Large: 5-15 mm). n=3

Figure 2. Comparison of Copper Adsorption onto Colonised and Non-Colonised Sweet Chestnut Charcoal of different particle sizes: Small: 0.5 - 2 mm; Medium: 2-5 mm; Large: 5-15 mm. (n=3).

Figure 3: Hygroscopic properties of charcoals produced from bamboo, oak and sweet chestnut at temperatures of 450, 650 and 850°C (n=3)

Figure 4: Survival of *P. fluorescens* in bamboo charcoal charred at 450, 650 or 850°C. Colonised charcoal particles were between 0.5 and 2 mm in size and were exposed to ambient air conditions of 20°C and 60% RH (n=3)

Figure 5: Survival of *P. fluorescens* in sweet chestnut charcoal charred at 450 or 650°C. Colonised charcoal particles were between 0.5 and 2 mm in size and were exposed to ambient air conditions of 20°C and 60% RH (n=3)

Figure 6: Survival of *P. jluorescens* in oak charcoal charred at 450, 650 or 850°C. Colonised charcoal particles were between 0.5 and 2 mm in size and were exposed to ambient air conditions of 20°C and 60% RH (n=3)

Figure 7: Survival of *P. fluorescens* in oak charcoal charred at 450, 650 or 850°C. Colonised charcoal particles were between 0.5 and 2 mm in size and were exposed to ambient air conditions of 20°C and 60% RH (n=3): Survival of *P. fluorescens* in sweet chestnut charcoal charred at 450°C over a three week period. Colonised charcoal particles were between 0.5 and 2 mm in size and were exposed held at 10°C and relative humidities between 0 and 100% (n=3)

Figure 8 Survival of *P. fluorescens* in sweet chestnut charcoal charred at 450°C over a three week period. Colonised charcoal particles were between 0.5 and 2 mm in size and were exposed held at 20°C and relative humidities between 0 and 100% (n=3)

Figure 9 Survival of *P. fluorescens* in sweet chestnut charcoal charred at 450°C over a three week period. Colonised charcoal particles were between 0.5 and 2 mm in size and were exposed held at 20°C and relative humidities between 0 and 100% (n=3)

Figure 10. Total number of bacteria (log cfu/g charcoal) colonising charcoal particles of ca 2mm in size that were impregnated with minerals and PAHs. All treatments were inoculated with a mixed bacterial suspension extracted from soil. The control treatment received no PAHs. (n=4)

Figure 11. Total number of bacteria (log cfu/g charcoal) colonising charcoal particles of ca 2mm in size that were impregnated with minerals and PAHs. All treatments were inoculated with a mixed bacterial suspension extracted from soil. The control treatment received no PAHs. (n=4)

Figure 12. Numbers (log cfu/g charcoal) of phenanthrene degrading bacteria compared with the total number of bacteria colonising charcoal particles of ca 2mm in size that were impregnated with minerals and phenanthrene only (conc. 20g phenanthrene $kg^{-1}$ charcoal). Charcaol particles were sampled weekly. The initial microbial inoculum consisted of a mixed bacterial suspension extracted from soil. (n=4)

Figure 13. Numbers (log cfu/g charcoal) of phenanthrene degrading bacteria compared with the total number of bacteria colonising charcoal particles of ca 2mm in size that were impregnated with minerals and phenanthrene and pyrene (conc. 10g phenanthrene and 10 g pyrene $kg^{-1}$ charcoal). Charcoal particles were sampled weekly. The initial microbial inoculum consisted of a mixed bacterial suspension extracted from soil. (n=4)

Figure 14: Carbon dioxide production from charcoal inoculated with microorganisms extracted from healthy soil, soil spiked with 1% (w/w) chicken manure and 5% (w/w) diesel oil and recycled charcoal. Controls that were used were not inoculated. (n=4).

Figure 15. Degradation of diesel oil in builder's sand that was amended with 5% (w/w) diesel oil. A non-charcoal amended control was compared with systems that were amended with different rates (5 and 10 (v/v) of inoculated and non-inoculated charcoal). (n=3).

Figure 16: Degradation rate of diesel oil in control treatment (no added diesel; 0 M.A.D). Respiration in flasks containing charcoal particles colonised by a mixed community of hydrocarbons (blue) was compared with non-inoculated charcoal (pink)(n=4).

Figure 17: Degradation rate of diesel oil in the treatment that contained 3.8 g diesel oil per 10 g charcoal (0.1 M.A.D). Respiration in flasks containing charcoal particles colonised by a mixed community of hydrocarbons (blue) was compared with non-inoculated charcoal (pink) (n=4). -

Figure 18: Degradation rate of diesel oil in the treatment that contained 7.6 g diesel oil per 10 g charcoal (0.2 M.A.D). Respiration in flasks containing charcoal particles colonised by a mixed community of hydrocarbons (blue) was compared with non-inoculated charcoal (pink) (n=4).

Figure 19: Degradation rate of diesel oil in the treatment that contained 11.4 g diesel oil per 10 g charcoal (0.3 M.A.D). Respiration in flasks containing charcoal particles colonised by a mixed community of hydrocarbons (blue) was compared with non-inoculated charcoal (pink) (n=4).

Figure 20: Degradation rate of diesel oil in the treatment that contained 15.2 g diesel oil per 10 g charcoal (0.4 M.A.D). Respiration in flasks containing charcoal particles colonised by a mixed community of hydrocarbons (blue) was compared with non-inoculated charcoal (pink) (n=4).

Figure 21: Degradation rate of diesel oil in the treatment that contained 19 g diesel oil per 10 g charcoal (0.5 M.A.D). Respiration in flasks containing charcoal particles colonised by a mixed community of hydrocarbons (blue) was compared with non-inoculated charcoal (pink) (n=4).

DETAILED DESCRIPTION OF THE INVENTION

The bacteria of the present invention are selected for their ability to metabolise the pollutant. It will be appreciated that certain, individual micro-organisms are known to catabolise certain pollutants, and charcoal containing such strains is included within the scope of the present invention.

A suitable source for bacterial isolates with specific metabolising properties is the National Collection of Industrial, Marine and Food Bacteria (**NCIMB** National Collections of Industrial, Marine and Food Bacteria, Ferguson Building, Craibstone Estate, Bucksburn, Aberdeen AB21 9YA Scotland: http://www.ncimb.co.uk).

Where such strains are used, it is preferred that the colonising bacteria not be restricted to just one of the following:

(a) bacteria of the genus *Nitrosomonas*; and/or
(b) bacteria of the genus *Nitrobacter*; and/or
(c) an *Azotobacter.*

The present invention selects strains from a naturally occurring environment, such as soil, sediments, standing water, groundwater, partially degraded organic matter, manure, sewage or similar materials present in sewage treatment plant, industrial effluents, industrial waste, sea water, soil, litter and/or waste streams.

Preferably, the material comprises a community of microbial strains isolated from a naturally occurring environment. Preferably, the strains are added to the charred material.

The communities are selected in the presence of an amount of the pollutant sufficient to preferentially select for at least tolerant microbes. The duration of cultivation and the concentration of pollutant may be readily determined by those skilled in the art. A preferred level is that which will kill or substantially slow down, or arrest, growth of non-tolerant organisms. Those organisms that are capable of metabolising a pollutant do not necessarily do better in the presence of the pollutant than in its absence, but should at least continue to reproduce, such that culturing a soil or water sample or extract will serve to select for such organisms. It will be apparent that organisms that thrive in the presence of the pollutant or any other selective carbon source will be preferably selected by such a technique, especially if the treated charcoal is incubated under conditions that put further selection pressure onto the colonising microbes.

It will be appreciated that, in order for the colonising bacteria or organisms to grow, or at least maintain their numbers, a carbon source will be required. This can either be derived from, or provided by, the target (polluted) site, can be present in the charcoal itself or added thereto, or can be provided by the substance. Suitable carbon sources will be readily apparent to the skilled person, and will be largely determined by the choice of the colonising bacteria. Non-tolerant i.e. non-colonising bacteria may be a suitable carbon source, at least initially.

Preferably, the charred material is sterile, as it will be immediately after charring, due to the elevated temperatures. The material can be retained in a sterile environment and preferably sealed, prior to inoculation with the colonising bacteria and the addition of the selected substance.

Preferably, the tolerant colonising organism is obtained from the polluted environment.

It will be readily apparent to the skilled person what levels of the pollutant are required in order to provide its selective effect. With some pollutants, only trace amounts may be necessary to exert a selection pressure, for instance pollutants that are highly toxic to at least the majority of the organisms that are present in the environment or could be expected to colonise the charred material, or at the very least, those organisms which compete with the colonising organisms or microbes of the present invention. With other, generally less toxic, pollutants, great amounts will be present to exert the selection pressure. A simple bioassay could establish such levels or they could be based on known toxicity levels for said pollutant, for instance in parts/unit volume or mass.

Inoculation may occur by a number of means and may include taking a sample of the affected area, or source material, comprising the pollutant and at least one organism tolerant thereof (and capable of metabolising or breaking down the pollutant) and adding the sample to the charred material.

Alternatively, the tolerant colonising organism may be selected by the user based on a knowledge or analysis of the target environment and is, therefore, preferably added to the material. This can be to "seed" the charred material, for instance.

The microbes may usefully be separated from the biological sample, such as the soil or sewerage etc, in which they occur, either straight away or when desired, such as after culturing in the presence of the pollutant. It may be preferable to incorporate a source of nutrients to accelerate the selection process.

Separation from the sample may be by any suitable means. One simple, and preferred, technique is to suspend a sample in water, for example, with agitation, and then allow the solids to settle out. The resulting supernatant, that can contain a large variety of microbial species, may then be used as the inoculum. If the sample is cultured, then a similar technique may be used. In either case, filtering and centrifugation may be employed, if desired. Alternatively, the culture may be allowed to stand, preferably in the presence of a selective amount of the pollutant it is desired to metabolise, and separation may simply be by harvesting any bio-film that is established.

The extracted organisms can then be introduced to a suitable charcoal. In general, the microbes will be suspended in an aqueous solution. The charcoal may be immersed in the suspension, or the suspension may be added drop-wise to the pre-treated charcoal, for example, allowing adsorption of the suspension between applications. A rapid method for achieving microbial infiltration of the charcoal is via vacuum infiltration where the treated charcoal is suspended in a microbial suspension and placed under vacuum. This results in the rapid replacement of any air within the charcoal by the microbial suspension.

As mentioned above, we have now discovered that it is possible to colonise charcoal with micro-organisms effective to metabolise a selected environmental pollutant, by seeding the material with the pollutant. This is distinct from the method of Takagi *et al.,* which claims to be effective in preventing contamination of groundwater via the creation of an enriched soil layer. However, their method does not prevent a range of (non-polluting) substances becoming adsorbed onto the charcoal. This non-selective adsorption of organic materials from the soil matrix will inevitably lead to the development of a microbial community that has only a small proportion of microbes of interest within its biofilm.

Secondly, the Takagi *et al.* method relies on natural adsorption processes of a pollutant from the soil matrix onto charcoal, so that minerals that have a low affinity to charcoal will be in short supply within the pore structure of the charcoal, thus limiting the development of the biofilm within the charcoal. Furthermore, their method is limited to organic pollutants that have a high affinity to charcoal. Because their method deals with creation of an enriched soil layer, they do not discuss the possibility of separating the charcoal from the soil.

JP 2005021748 discloses carbon powder comprising 'nutrition sources for soil bacteria.' However, there is no suggestion that charcoal, together with bacteria and a pollutant, should be mixed prior to use of charcoal in a target area. Similar considerations apply to JP 8001133.

JP 8252086 describes a porous metal colonised with bacteria, but there is no suggestion of selection of the organism or microbe based on the pollutant. DE 19928087 describes activated charcoal as a carrier matrix for microorganisms. JP 11104600 relates to making charcoal and then using it to grow lactobacillus.

JP8309375 and all of the above are also silent on selection. However, by seeding the charred material with the pollutant, we create an environment within the charcoal that provides selective growth conditions for organisms that are capable of degrading the pollutant that is seeded into the charcoal. The resulting material is colonised by microbes capable of degrading the selected environmental pollutant(s). In other words, the growth conditions created in the charred material select for the very organisms that are most useful in bioremediation of the selected pollutant.

Preferably, minerals are added directly to the charcoal. The advantage of this is that the development of a pollutant degrading community within the charcoal is not limited by the ability of the charcoal to adsorb minerals from the soil matrix.

It is also preferred that the charcoal is seeded directly with an environmental pollutant of concern (or a mixture thereof). This is advantageous because the effect of the colonised material is not limited to the degradation of pollutants with a high affinity to charcoal.

A further advantage of the present invention is that, in some embodiments, it circumvents the mixing of clean charcoal into (polluted) soil but creates instead a soil-free charcoal product that contains large populations of organisms with metabolic capacities that can be adjusted at will by changing the selection pressures within the charcoal.

Therefore, the microbially colonised charcoal products allow prolonged survival of the microbial community within the charcoal, even under conditions that would normally lead to rapid cell death.

Preferably, charcoals with small macro pore sizes, preferably in the region of about 5 - 15$\mu$m are provided. Preferably, the charcoal has hygroscopic capacities, that further prolong microbial survival. Such charcoals can typically attract >0.3% of their weight in water from air with a relative humidity of 60%.

Whereas environmental pollutants are normally preferred to select for organisms that degrade such substances, it is possible to use at least one selected organic substrate, or any combination thereof, selected inhibitors or selected growth conditions to select for microbes with a desired functional trait. Therefore, besides encouraging colonisation with pollutant degrading bacteria, charcoal can become colonised with microbes that can be used to control odours, control pathogens and pests, provide plant growth promotion, fix atmospheric nitrogen, oxidise ammonia and nitrites, or are beneficial to human health.

It is preferred that colonisation of the charcoal further comprises a period of incubation of the impregnated charcoal, so as to allow a bio-film to form and to degrade a majority of the pollutant that has been used to select for organisms with a specific metabolic trait, such as the ability to degrade a specific pollutant.

Incubation and formation of a bio-film is greatly enhanced by the presence of suitable metabolites and/or nutrients. These may be present in the microbial suspension used to inoculate the charcoal. However, it is generally acceptable to pre-treat the charcoal with one or more salts and/or vitamins and/or trace elements. Where the pollutant is not able to provide either carbon or nitrogen, or both, in sufficient quantities, then suitable nutrients comprising these may also be added. Where salts or minerals are referred to herein, then these preferably comprise one or more of $K_2HPO_4$, $KH_2PO_4$, $(NH_4)_2SO_4$ and NaCl. Other useful mineral nutrients include one or more of: $FeSO_4$, $ZnSO_4$, $MnCl_2$, $CoCl_2$, $CuCl_2$, $NiCl_2$, $NaMoO_4$, $H_3BO_4$, a Mg salt, and a Ca salt.

Other nutrients preferably comprise one or more of biotin, folic acid, thiamin HCl, D-calcium pantothenate, vitamin $B_{12}$, riboflavin, niacin, pyridoxal HCl, and para-amino benzoic acid.

Therefore, the material preferably has been pre-treated with:

i) at least one salt, such as one or more of $K_2HPO_4$, $KH_2PO_4$, $(NH_4)_2SO_4$ and NaCl; and/or
ii) at least one mineral nutrient, such as one or more of: $FeSO_4$, $ZnSO_4$, $MnCl_2$, $CoCl_2$, $CuCl_2$, $NiCl_2$, $NaMoO_4$, $H_3BO_4$, a Mg salt, and a Ca salt; and/or
iii) at least one nutrient, such as one or more of: biotin, folic acid, thiamin HCl, D-calcium pantothenate, vitamin $B_{12}$, riboflavin, niacin, pyridoxal HCl, and para-amino benzoic acid; and/or a carbon and/or nitrogen source.

It is also generally preferred that the material comprises an organic nutrients source to which a selective pollutant is added such as one or more antibiotics, heavy metals or toxins.

Preferably that the material has been pre-treated with:

i) at least one mineral nutrient, such as one or more of $K_2HPO_4$, $KH_2PO_4$, $(NH_4)_2SO_4$ and NaCl, $FeSO_4$, $ZnSO_4$, $MnCl_2$, $CoCl_2$, $CuCl_2$, $NiCl_2$, $NaMoO_4$, $H_3BO_4$, a Mg salt, and a Ca salt;
iii) at least one vitamin, such as one or more of: biotin, folic acid, thiamin HCl, D-calcium pantothenate, vitamin $B_{12}$, riboflavin, niacin, pyridoxal HCl, and para-amino benzoic acid; and/or a carbon and/or nitrogen source;
iii) an organic nutrient source; and/or
iv) an organic nutrients source to which a selective substance is added such as one or more antibiotics, heavy metals or toxins.

In order that the community in the charcoal remains selective for a specific carbon source or pollutant, if the pollutant is not a carbon source, a selective level of a specific carbon source is provided. This may be added before, after, or with the salts/vitamins/trace elements. However, as the salts will generally be administered to the charcoal in the form of an aqueous solution, then it is generally preferred to add the pollutants separately, as this will generally be administered in an organic solvent, given the relative hydrophobicity of most pollutants. Water soluble carbon and nitrogen sources are normally administered together with the minerals and vitamins. If a solvent is used, it is generally preferred to remove the solvents from the charcoal prior to impregnation with the subsequent solution, especially where the solutions are not readily miscible.

Seeding the charcoal used in the method of the invention in this way ensures that the microbial community introduced into the charcoal stays true, and has significant advantages over the art, where charcoal is added to a site containing a contaminant and where the charcoal picks up all of the local contaminants with any associated bacteria. Using the present invention, a particular environmental substance or compound can be targeted and samples of bacterial cultures taken from different sources to establish a desirable microbial community, and any serendipitous element is minimised.

Although the various pre-treatment ingredients may be generally impregnated into the charcoal using solutions, any other means is acceptable, including condensation or impregnation with fine dusts, for example.

It is also possible to inoculate the charcoal prior to administering with further ingredients or pollutant, but it is then necessary to ensure survival of the microbial community associated with the charcoal, which can preclude the use of heat to drive off solvents and, despite the fact that the microbial community is intended for the metabolism of certain pollutants, organic solvents may still not be compatible with the microbial community.

Thus, the selected pollutant can be added to the material by any suitable method, including coating, inoculating, painting, spraying, impregnating or dunking of the material in a solution of the selected substance.

Besides nutrients and a selective quantity of pollutant, other materials can be added to the charcoal that would boost the metabolism of the microbial community. This is especially useful when the bacteria require extra electron acceptors for the effective oxidation of certain organic pollutants. Such materials include, peroxides, nitrates, oxygen, and sulphates among others.

Alternatively, when the pollutants are chlorinated hydrocarbons, for instance, reducing agents, such as elementary iron, chromium or metal sulphides among others, could be added to the charcoal.

Thus, it is preferred that nutrients suitable for the colonising organisms and/or oxidising or reducing agents are also added to the charred material. The choice of such nutrients, oxidising or reducing agents, will be apparent to the skilled person based on the organism and the selected pollutant to be degraded or transformed and the presence of otherwise of the nutrients, oxidising or reducing agents at the target or affected area, i.e. *in situ.*

Further communities may be isolated in a similar manner to that given above, but it will generally be preferable to establish a community in the form of a culture which can then be perpetuated, preferably by cultivation under selective conditions. This perpetual culture may then be used to inoculate as much charcoal as desired with a selected community of microbes.

In the alternative, cultures for further inoculation of charcoal used in the method may simply be obtained from charcoal of the invention that has already been colonised. This may be achieved, for example, by grinding up colonised charcoal and incubating a suspension obtained therefrom with charcoal ready for inoculation.

In one aspect, un-inoculated charcoal is added to a site containing colonised charcoal of the invention to provide an additional stable growing environment for the colonising bacteria. It is preferred that the un-inoculated charcoal contains a selective amount of the substance to be metabolised. In a preferred embodiment, the process is perpetuated by the addition of: a) further nutrients, and/or b) further colonised charcoal and/or c) further uncolonised charcoal.

The uncolonised charcoal may be plain charcoal that has not been pre-treated. However, it is preferred that this charcoal has been pre-treated with nutrients, especially salts, but which may also, alternatively or in addition, comprise other nutrients such as vitamins and sources of carbon and/or nitrogen. More preferably, there is provided charcoal suitable for use in the method in accordance with the present invention, and which has been pre-treated with at least one nutrient and a selective level of pollutant. In this respect, a selective level is that which will compromise growth of a majority of organisms not tolerant of the pollutant.

It will be appreciated that the colonised charcoal used in the method of the present invention may be effective against more than one pollutant. In addition, other beneficial micro-organisms, and especially bacteria (*vide infra),* may be used to colonise the charcoal.

Particularly preferred pollutants may be selected from hydrocarbons, cyanides, nitrates, ammonia, and so forth. Preferably, the pollutant is degradable or can be transformed into a non-toxic or insoluble product via microbial action. In this respect pollutants can be oxidised, reduced, or be combined with microbial products or biomass to form insoluble products. More preferably, the pollutant is selected from the group consisting of polycyclic aromatic hydrocarbons (PAHs), benzene, toluene, ethylbenzene, xylenes, pyrene and phenanthrene, trichloroethylene, phenols, and chlorinated phenols.

Therefore, the material can be thought of as a bio-catalyst, as it provides a locus for conversion of a pollutant to a its break-down products.

Other preferred selected substances include radio isotopes, oxides of Nitrogen or Sulphur and Heavy Metals. In respect of these contaminants, it will be appreciated that a carbon source may be required, depending on the growth conditions required and the nature of the colonising microbes themselves. It will also be appreciated that, in respect of substances such as radio isotopes and heavy metals, in particular, the organisms will "metabolise" the substance in the sense of remove it from the environment or subject the substance to a biological process that leads to a reduction of its concentration or activity (for instance the potency or poisonous, carcinogenic or harmful effect of the substance) at the environmental site for bioremediation.

For the re-use, or re-cycling, of the microbially colonised charcoal used in the method of the present invention, further administration of essential nutrients, oxidising agents and/or reducing agents will generally be required where these are not available, or are only available in limited quantity, from the environment in which the pollutant is located.

The nature of the charcoal used in the method will be determined by the use to which the colonised charcoal is to be put. In general, it is preferred that the charcoal be sufficiently robust so as not to disintegrate in the environment in which it is to be used. Thus, although most charcoals can be used in the present invention, the more robust charcoals, such as those from various wood sources, are preferred. This type of material is especially favoured in cases where storage of the material at low relative humidity is required. Thus, wood and bamboo charcoals are particularly preferred, but charcoals from relatively fragile sources, such as straw, and nettles, may be used, especially where the charcoal is bounded by a retention means, such as muslin or gauze, or some other form of barrier preventing the free movement of the charcoal within the environment in which it is to be used. An example might be a sock containing the charcoal being suspended in contaminated water or air.

The size of the charcoal used does not appear to greatly affect the rate of colonisation, so that preparation of the charcoal is not a significant consideration in the selection of the size of pellet/briquette used to treat the occurrence of pollutants. However, one particular advantage of the charcoal is that it serves to adsorb heavy metals and other potential toxins onto its surface, thus preventing such compounds having a deleterious effect on the bacteria associated with the charcoal. This effect can be further enhanced by the colonising organisms that will produce biofilms within the charcoal where the biofilm itself provides further protection against chemical stressors or stress agents. Such stressors may be chemical agents such as heavy metals, cyanides, salts, disinfectants, antibiotics or pesticides that would normally harm the colonising bacteria if they were directly exposed to them. However, metabolic break down products of the pollutants may be excluded from this definition, at least at low concentrations.

Where there is a relatively large amount of heavy metals and other toxicants present, then it will generally be preferable to use larger charcoal particles in order to maximise the protection of the bacteria.

It will be appreciated that fungi are also preferred as the organism. Fungal hyphae have on average a thickness of between 5 and 10 $\mu$m and the macro-pores of most wood charcoals will be able to accommodate this size of hyphae. In exceptional circumstances hyphae can be as thin as 1 $\mu$m or as thick as 30 $\mu$m. In the case of using charcoal to become colonised by fungi with thick hyphae, it is appreciated that charcoals should be used with very large pore diameters such as those found in charcoal made from bamboo.

In general, where reference is made to bacteria, this also includes fungi, unless otherwise apparent.

Where charcoal is powdered, then it will be appreciated that the powder should only be coarse, in order to allow colonisation by bacteria, so that a minimum diameter of about 0.1mm is generally preferred.

Larger pellets may be required for use in liquid environments, in order not to impede flow, while smaller pellets may be appropriate where a brown-field site is being treated.

The only limit on the nature of the charcoal is as to whether it is colonisable by suitable microbes. In particular, the charcoal preferably has a tubular structure with an average tube diameter of >2 $\mu$m. Unless there is a particular reason for using activated charcoal, then it is generally preferred not to use activated charcoal, as the procedure for making it is generally more expensive than that for making non-activated charcoal, for example wood charcoal.

The internal macro-pore surface area of wood charcoal is approximately 4 m$^2$/g charcoal, and colonisation rates of 10$^9$ - 10$^{10}$ bacteria per g charcoal are easily obtained. Once the charcoal has been prepared, it may be stored. In this respect, charcoal is generally hygroscopic. Thus, provided that the charcoal is not kept in hermetically sealed packs, it can generally serve to attract water to keep at least some level of hydration for the colonising bacteria. As a general rule, the higher the temperature of the initial charcoal manufacture, the greater the hygroscopic potential of the charcoal is. Furthermore, charcoals with smaller macro-pore sizes normally provide better survival conditions for the colonising microbes than charcoals with large pores.

The level of colonisation of the charcoal will depend on a number of factors but it may be desirable to colonise the charcoal to its upper limit. In such a case, further growth of the colony will not be possible within the charcoal, but may allow colonisation of the environment and processing of the pollutant. If the pollutant is not an actual nutrient of the colony, then this can be advantageous.

Where the pollutant is a nutrient for the colony, then it is generally preferable to colonise the charcoal to a level at least two orders of magnitude below that at which it is fully colonised, in order to permit substantial growth in the target environment. This also permits free movement of pollutants into the charcoal from the surrounding environment.

The colonised charcoal used in the method of the present invention may be used in any circumstances where it is desired to reduce the levels of a pollutant and where it is possible for the microbes to act. In a brown field site, for example, colonised charcoal of the invention may be mulched into the soil and, once the soil is cleared of the pollutant, the resulting soil can be used as compost, with the charcoal acting a source of delayed release nutrients.

The charcoal used in the method of the invention may also be used in the form of pellets in the treatment of liquid waste, such as slurries or sewage, or for the treatment of contaminated surface or ground water, for example. Typically, the pellets will be held in containers, such as baskets or socks and suspended in the liquid which may be either moving or still. It will be appreciated that a level of movement within the liquid will generally serve to accelerate removal of the pollutants.

In a further embodiment, the charcoal of the invention may be provided as part of an absorbent structure, such as where the charcoal is sandwiched between layers of absorbent material, such as cloth or paper. The resulting structure may be provided as sheets which can be used to mop up and degrade fuel or oil spills on hard surfaces, for example. The sheets may then be removed and placed in a suitable environment to allow the bacteria to degrade the pollutant absorbed by the sheets, or may be left in place, if the environment is suitable, if there is sufficient moisture present, for example. In such a case, sheets can be left until they start to disintegrate or become ineffective. Sheets removed for processing may be placed in a container with some water, for example, to allow rapid degradation of the adsorbed hydrocarbons before the material is disposed of or recycled

Charcoal used in the method of the invention may also be provided in floating mattresses or booms, for example, which are particularly useful to adsorb and degrade oil spills at sea.

Where the charcoal used in the method of the invention is introduced to a site without any intention of removing the charcoal for rejuvenation, then it may be desirable to add further nutrients to the site to encourage growth of the microbes within the charcoal, thereby to assist in removing the pollutant. This can include soil tillage, addition of compost, culturing of plants, etc all of which would further improve soil conditions and therefore enhance the activity of the charcoal of the present invention. Such measures can be applied either as a pre-treatment, or a co-treatment while there is an expectation that the microbes remain effective.

It will be understood that adsorption of pollutants onto the charcoal from the environment, and subsequent degradation of the pollutant by the colonising microbial community within the charcoal, is a likely decontamination route.

However, it will also be appreciated that microbes that are brought into the environment in charcoal particles are likely to be able to colonise the environment outside the charcoal, as there is, in some embodiments, no physical barrier that would prevent such colonisation. This ability of microbes to colonise the wider environment using the colonised charcoal as the inoculum source is especially useful in cases where mixing of the charcoal into the polluted environment is limited or where further colonisation of the wider environment is a pre-requisite, as is the case with biological control or the oral administration of pro-biotic organisms.

In other forms of treatment, especially in liquids, charcoal pellets may be removed and rejuvenated. Rejuvenation may take any suitable form. For example, it may be that there is a considerable amount of heavy metal contamination, and it may be desirable to use chelating agents, for example, to remove heavy metals from the charcoal that has been exposed to the polluted environment. Rejuvenation may also comprise, for example, elevating the temperature of the charcoal to encourage metabolism of the pollutant, and will also typically comprise further dosing with minerals and/or nutrients. The rejuvenated charcoal can then be used once again, in the same or another environment, and this process may be repeated as often as is desired.

The methods of the present invention are useful in the treatment of selected compounds that are present in fluids, in the atmosphere, or bound to solids such as a soil minerals and organic matter. Examples include contaminated soils both *in situ* and *ex situ* (including brown field sites, for example), water, sewage, sewage sludge, oil waste (such as bottom sludge), oil spills, petroleum spills, landfill, landfill leachate, biodegradable waste, industrial discharge, and airborne contaminants.

The present invention can be thought of as the provision of a catalyst for bioremediation or de-contamination of an environment, from a selected substance.

For example, the present invention can degrade short chain fatty acids, hydrocarbons and ammonia.

Preferably, the pollutant is Ammonia, Ammonium compounds, or salts or derivatives thereof. Preferably, the organism is, therefore, selected from Nitrosomonas spp. and Nitrobacter spp which oxidise ammonium to nitrites and nitrates respectively.

The charcoal bioreactors will naturally adsorb these collutants where they are subsequently degraded by the bacteria associated with the charcoal. In fact, the charcoal can additionally be used as a carrier material for a variety of beneficial organisms that are to be introduced into the environment such as bacteria and fungi that are used to control plant pests and diseases (biological control organisms), nitrogen fixing bacteria such as *Rhizobium* spp and plant growth promoting organisms such as *Pseudomonas* spp., as well as introduction of micro-organisms which will enhance the degradation of biodegradable wastes e.g. municipal solid waste, green waste; or remediation of contaminants e.g. hydrocarbons contained in exhaust or chimney gases.

In addition, the charcoal can be used as a carrier of health promoting organisms such as *Lactobacillus* species, including: *L acidophilus, L amylovorus, L. brevis, L. casei, L casei* subsp. *rhamnosus (Lactobacillus GG), L. caucasicus, L. crispatus, L. delbrueckii* subsp. *bulgaricus (L. bulgaricus), L. fermentum (L fermenti), L. gasseri, L. helveticus, L johnsonii, L. lactis, L. leichmannii, L. paracasei, L. plantarum, L. reuteri, L. rhamnosus, Bifidobacterium* species including *B. adolescents, B. bifidum, B. breve, B. infantis, B. lactis (B. animalis), B. licheniformis, B. longuim* other lactic acid bacteria such as *Enterococcus faecium, Lactococcus lactis, Leuconstoc mesenteroides, Pediococcus acidilactici, Streptococcus thermophilus* and non-lactic acid bacteria such as *Bacillus subtilis, Escherichia coli strain nissle, Saccharomyces boulardii* and *Saccharomyces cerevisia.*

In general it is preferred that further selection of microbial communities or populations with desirable traits, such as tolerance to pollutants, can be achieved by incubating the material as described herein under conditions that specifically encourage the growth of the desired microbial community.

Preferably, the selective pollutant may be selected from the group consisting of: at least one carbon source, at least one nitrogen source, at least one antibiotic, at least one cell toxin, at least one heavy metal, at least one mineral, and at least one vitamin.

Preferably, the microbes are selected from the group consisting of: hydrocarbon degraders (such as *Arthrobacter, Actinobacter, Pseudomonas* spp, *Mycobacteria, Rhodococcus* spp, *Spingomonas* as well as fungi that produce peroxides and laccases that oxidise aromatic ring structures found in many pollutants), biological control agents (such as Pseudomonas spp, Bacillus spp, and a great many types of biological control fungi), plant growth promoting organisms (such as *Pseudomonas fluorescens* and mycorrihiza), nitrogen fixers (such as *Rhizobium* spp, Frankia, Azotobacter, Anabeana, Nostoc, Azotobacter, *Beijerinckia* and *Kiebsiella* among others) and health-promoting organisms selected from Lactobacilli, *Bifidohacterium, Enterococcus faecium, Lactococcus lactis, Leuconstoc mesenteroides, Pediococcus acidilactici, Streptococcus thermophilus* and non-lactic acid bacteria such as *Bacillus subtilis, Escherichia coli strain nissle, Saccharomyces boulardii* and *Saccharomyces cerevisia*)

Preferably, a population of the microbes is provided in macro-pores of between 5 and 15 $\mu$m. Preferably, the pore structure provides sufficient protection from desiccation to thereby allow prolonged survival of a microbial population therein.

It is also preferred that the charcoal is sufficiently hygroscopic so that the microbial community within the charcoal can obtain sufficient moisture to allow prolonged survival.

As discussed above, it is also preferred that the microbial population within the charcoal creates a biofilm that protects the microbes within that charcoal from desiccation. Preferably, the microbial population within the charcoal creates a biofilm that protects the microbes within that charcoal from chemical stress.

**Example 1**

**Conditioning of charcoal particles to encourage growth and establishment of a specific microbial communities within.**

**Introduction**

Charring of organic matter, results in the removal of most O, N and H groups from the material, leaving a material that consists mainly of carbon and mineral groups that cannot be removed by heating alone. When exposed to oxidising conditions, some oxidation of carbon groups within the charcoal will take place, making the charcoal more 'reactive'. The deliberate oxidation of charcoals using steam, high temperatures and oxidising materials is known as 'activation'. Microbial oxidation of the carbon groups in charcoal is however a very slow process that might take hundreds of years under normal conditions. It is therefore not surprising that charcoal that was formed many thousands of years ago can be recovered more or less intact from soil. For all intents and purposes it can be stated that charcoal is biologically inert and can not be used as a energy, nutrient or mineral source by bacteria. When brought into soil, charcoal will become colonised by micro-organisms as minerals and degradable organic substances will accumulate within the charcoal allowing the development of a <u>nonspecific</u> microbial community within.

However, in order to allow profuse colonisation of charcoal particles by a <u>specific</u> microbial community it is necessary to 'charge' the charcoal with both minerals and a specific organic substrate. Whereas the formulation of the mineral additions could be adapted to suit specific microbes, we have opted for a mineral medium that is 'complete' in that it contains a complete set of minerals and vitamins, even those that can be synthesised by some bacteria. To create a 'selective' environment for specific hydrocarbon and cyanide degraders in addition to this medium a carbon source is added, which can be a pure substrate or a mixture of substrates.

<u>Mineral and vitamin solution used to 'charge' the charcoal</u>

A basal salt medium was used containing per litre of water:

0.4 g $K_2HPO_4$
0.4 g $KH_2PO_4$
0.4 g $(NH_4)_2SO_4$
0.3 g NaCl

This solution was autoclaved. Once cool, the following was added per 985ml:

5 ml filter sterilised vitamin solution (see Table 1);
5 ml filter sterilised Trace element solution (see Table 1); and
5ml Mg and Ca solution.

The Mg and Ca solution contains per litre of water 400 mg $MgSO_4.7H_2O$ (Fisons plc, UK) and 400 mg $CaCl_2.2H_2O$ (BDH).

**Table 1:** Vitamins and trace elements for Basal Salt Medium (BSM)

| Vitamins | | | Trace elements | | |
|---|---|---|---|---|---|
| Compound | mg/l | | Compound | mg/l | |
| Biocin | 20 | | $FeSO_4.7H_2O$ | 200 | |
| Folic Acid | 20 | | $ZnSO_4.7H_2O$ | 10 | |
| Thiamin HCl | 50 | | $MnCl_2.4H_2O$ | 3 | |
| D-calcium pantothenate | 50 | | $CoCl_2.6H_2O$ | 20 | |

(continued)

| Vitamins | | | Trace elements | | |
|---|---|---|---|---|---|
| Compound | mg/l | | Compound | mg/l | |
| Vitamin $B_{12}$ | 50 | | $CuCl_2.2H_2O$ | 1 | |
| Riboflavin | 50 | | $NiCl_2.6H_2O$ | 2 | |
| Niacin | 200 | | $NaMoO_4.2H_2O$ | 500 | |
| Pyridoxal HCl | 30 | | $H_3BO_4$ | 30 | |
| Para-aminobenzoic acid | 20 | | | | |

**Example 2**

**Bacterial colonisation of different sized charcoal pieces.**

**Introduction**

It was hypothesized that if both internal and external surfaces of a charcoal particle are available for microbial colonisation, different sized particles would support the same number of bacteria per unit weight. On the other hand if part of the internal structure of a charcoal particle would be unavailable for microbial colonisation, smaller particles will support a greater microbial population than larger particles.

**Methodology**

To estimate if the pore structure within charcoal is continuous, thus allowing complete colonisation, of its internal structure, sweet chestnut wood was charred at 450°C, broken up into pieces, and graded by passing over a nest of sieves to obtain pieces with the following diameter classes: Fine: <0.5 mm; Small: 0.5 - 2 mm; Medium: 2-5 mm; Large: 5-15 mm. Subsequently, from each class size, three, I g samples were taken and soaked in TSB (tryptic soy broth) till saturation. After draining excess liquid from the sample, each sample was mixed with approx. $10^5$ *Pseudomonas fluorescens* bacteria and the sample was subsequently incubated for one week to allow colonisation of the available charcoal surfaces. After incubation, the charcoal was rinsed for 24 h in sterile RO water to remove non-attached bacteria.
To estimate the level of bacterial colonisation, each sample was suspended in 9 ml ¼ strength Ringer's solution and crushed in a sterile pestle and mortar before a 10 fold dilution series was prepared. Each dilution was subsequently spread plated onto 10% strength TSA and plates were incubated for 48 hours before plates containing between 20 and 100 colonies were counted. From these counts colonisation of the different charcoal size classes was calculated.

**Results**

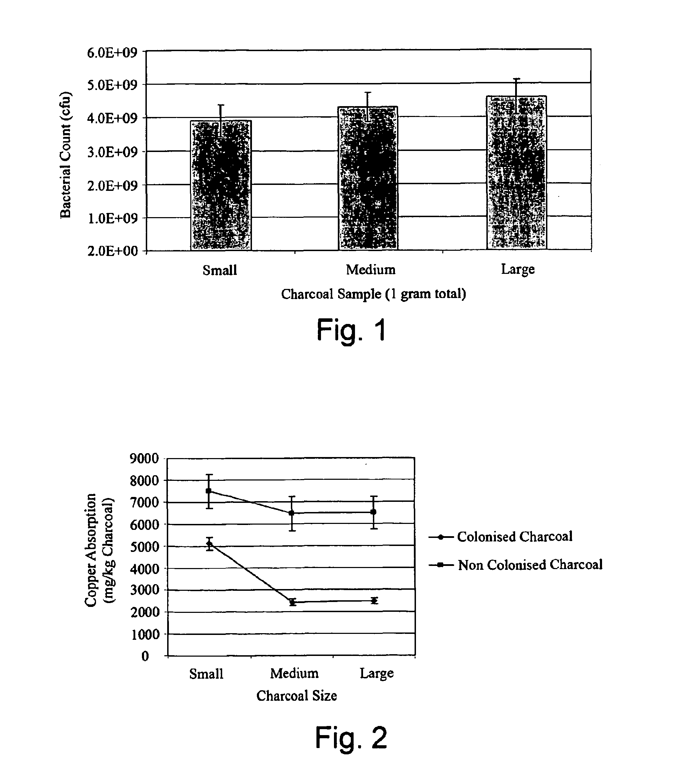

Similar numbers of bacteria (differences were not significant at $P<0.05$) colonised the different sized charcoal particles suggesting that the internal surfaces within the charcoal were all available for microbial colonisation (Fig. 1).

**Conclusions**

o Wood charcoal impregnated with a suitable microbial growth medium can become colonised with a large population of microbes (> $5 \times 10^9$ bacteria per g charcoal)
o Colonisation potential of charcoal pieces (expressed as no. of bacteria per g charcoal) is not significantly affected by the size of the charcoal pieces, suggesting that a charcoal particle consists of continuous pores.

**Example 3**

**Protection of microbes against chemical stress in charcoal.**

**Introduction**

Once brought into a hostile environment, survival of bacteria within charcoal particles will be dependant on the ability of the charcoal, or the bacterial community colonising the charcoal, to protect the colonising community against chemical stressors.

It was hypothesised that bacteria forming a biofilm onto the internal surfaces of a charcoal particle will inhibit the adsorption of metal ions to some extent. The reason for this is two fold. Firstly, polysaccharides produced by bacteria do not bind metals as effectively as charcoal surfaces themselves, and secondly, bacterial colonisation of the internal structure of a charcoal particle will lead to pores becoming blocked thus preventing metals from entering these pores. If the former mechanism (surface cover by polysaccharides) is the most important mechanism preventing metal adsorption, it would be expected that particles with different sizes and similar microbial colonisation (per g charcoal) would adsorb similar levels of metal ions. On the other hand, if pore blockage as a result of microbial colonisation would have been an issue, larger particles would adsorb less metal than smaller ones. A reduction of metal adsorption onto charcoals would suggest that charcoals colonised by microbes would be protected from chemical stresses making them more suitable to be added to soils that are contaminated with both hydrocarbons and metals.

**Methodology**

Charcoal particles (obtained by charring sweet chestnut wood at 450°C) of size classes of 0.5 - 2 mm (small), 2-5 mm (medium) and 5-15 mm (large) were chosen to test the above hypothesis. Subsequently, from each class size, three, 1 g (dry weight) samples were taken and soaked in TSB (tryptic soy broth) till saturation. After draining excess liquid from the sample, each sample was mixed with approx. $10^5$ *Pseudomonas fluorescens* bacteria and the sample was subsequently incubated for one week to allow colonisation of the available charcoal surfaces. After incubation, the charcoal was rinsed three times in sterile RO water to remove non-attached bacteria. Subsequently each colonised charcoal sample (0.5 g dryweight) was added to a 250 ml solution containing 250 ppm $CuSO_4$. Non-colonised samples were used for comparison. To allow maximum metal adsorption, colonised and non-colonised samples were incubated for 72 hours on a shaker at 100 rpm. Subsequently the charcoal was removed via filtration over a Watman no. 1 filter paper, ashed and digested in concentrated nitric acid. Copper adsorbed onto each sample was estimated using Atomic Adsorption (AA)

**Results**

Charcoal particle size had no significant effect on the ability of non-colonised charcoal to adsorb Cu ions from a solution, suggesting that the time allowed for adsorption was sufficient to reach the inner most pores of the larger charcoal particles. Bacterial colonisation reduced the ability of charcoal to adsorb metals by 30% when the charcoal particles were small. Charcoal particle size had a further significant effect on Cu adsorption with large and medium sized colonised particles adsorbing only 50% of the amount of Cu compared to the small colonised particles (Fig. 2). This suggests that bacterial colonisation leads to blockage of pores and that this effect is of lesser importance when the charcoal particles are small (< 2 mm). Larger particles on the other hand afford more protection for bacteria against toxic effects of, for example, heavy metals.

**Conclusions**

○ The bacterial community within charcoal particles will provide protection against chemical stress.
○ Larger charcoal particles will provide more protection against chemical stress for the colonising microbes.

**Example 4**

**Survival of bacteria in wood charcoal exposed to physical stress**

**Introduction**

To be able to develop effective products based on charcoal colonised by a community of hydrocarbon degraders, it is essential that the charcoal provides sufficient protection to the colonising bacteria from desiccation. This is especially important if the product is to be stored for extended periods of time or brought into an environment that is hostile. Normally, bacteria (except for endospores) survive poorly (less than 1 day) under ambient air conditions of low relative humidity and temperatures of >20°C.

Bamboo charcoal is known to be hygroscopic (US patent 5918333) and this property alone could enhance the survival of bacteria colonising charcoal. Furthermore, the temperature at which charring takes place is known to affect the hygroscopic properties of charcoal, with higher charring temperatures normally resulting in charcoal that is more hygroscopic. Besides hygroscopic properties of charcoal, pore size is likely to affect bacterial survival, where smaller pores could afford better microbial survival.

**Methodology**

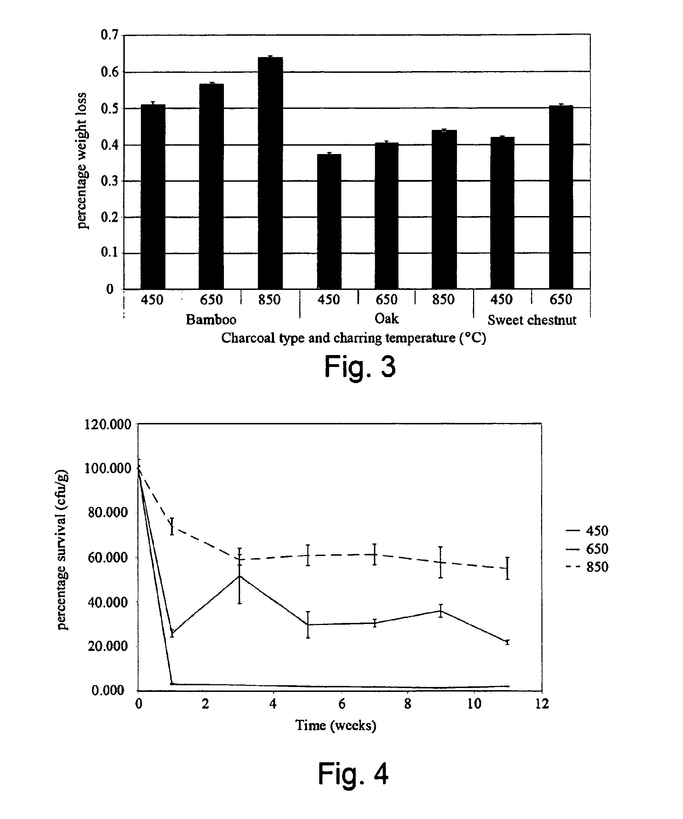

To test microbial survival in charcoal, a range of charcoals produced from different woody materials (oak, sweet chestnut and bamboo) were charred at 3 different temperatures (450, 650 and 850°C). Using SEM, Bamboo was found to have an average macro-pore size of around 25 $\mu$m; sweet chestnut pores were around 15$\mu$m, while the pores in oak wood were around 12 $\mu$m. These different source materials showed significant differences in hygroscopic properties with bamboo charcoal being 40% more hygroscopic than charcoals produced from either sweet chestnut or oak wood. Charring at 850°C resulted in charcoals that were around 20% more hygroscopic than charcoals produced at 450°C (Fig. 3).

To test the effects of pore size and hygroscopic properties of the different charcoals on bacterial survival, an isolate of *Pseudomonas fluorescens* (a Gram negative non-spore forming bacterium) was used to inoculate charcoal particles that had a diameter of between 0.5 and 2 mm. These particles were vacuum infiltrated with tryptone soya broth (TSB). Subsequently half the volume of TSB inside the charcoal was evaporated at 60°C before 60 g of charcoal was inoculated with a 100ml suspension of *P. fluorescens* containing around $10^7$ bacteria per ml. The thus inoculated charcoals were placed in a laminar flow cabinet for several days to allow bacterial colonisation of the charcoal and and to allow removal of all free liquid. Whereas colonisation density varied between different charcoals, bacterial numbers were in general between $10^8$ and $10^9$ live bacteria per g charcoal (dry-weight). After inoculation, batches of 2 g charcoal were placed in open universal bottles and exposed to a variety of environmental conditions. Survival of the bacteria was quantified by pour plating a dilution series of finely ground charcoal obtained from the different treatments in TSA (Tryptone soya agar).

Two experiments were carried out to assess bacterial survival. In the first experiment the long term survival of bacteria in three different charcoals, charred at 450, 650 or 850°C was quantified under ambient conditions of 20°C and 60% Relative humidity (RH). Each data point was repeated in triplicate using separate batches of charcoal and survival was monitored over an 11 week period (T0, T1, T2, T3, T4, T5, T6, T7, T9 and T11 weeks). Because there was considerable variation in absolute microbial numbers in the different charcoals, survival was expressed as percentage survival, taking the count at T0 as 100% survival.

In a second experiment colonised sweet chestnut charcoal (charred at 450°C) was exposed to 10, 20 or 30°C at a RH of 0, 60 or 100% giving a total of 9 treatments. As in the previous experiment three separate batches of charcoal were sampled for each data point. Survival was monitored over a three week period (T0, T1, T2 and T3 weeks) and results were expressed a numbers of bacteria per g charcoal.

**Results**

**Experiment 1: Long term survival of bacteria in charcoal at ambient conditions of 20°C and 60% RH.**

Survival of bacteria in charcoal at ambient conditions was surprisingly good in all charcoals, except bamboo charcoal charred at 450°C where bacterial numbers dropped by two log-units (to 1% of the original inoculum) during the first week (Fig. 4). Bamboo charcoal charred at higher temperatures provided conditions that gave survival rates of around 40% for bamboo charcoal charred at 650°C and 60% for bamboo charcoal charred at 850°C over the duration of the experiment. (Fig. 4).

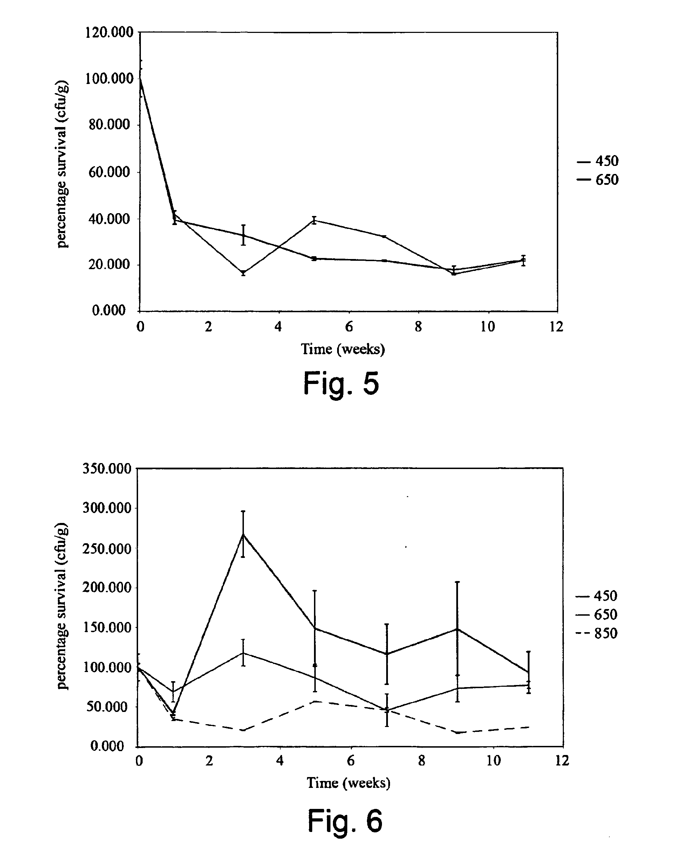

Microbial survival in sweet chestnut charcoal over the 11 week period dropped to around 20% of the original bacterial inoculum. There was no significant effect of charring temperature on survival rates in sweet chestnut charcoal. (Fig. 5).

Survival rates of *P. fluorescens* in charcoal produced from oak wood at different temperatures was very high, with survival rates between 50 and 100%. (Fig. 6).

**Conclusions**

○ Charcoals produced from woody materials create excellent survival conditions tor bacteria, allowing inoculated wood charcoal to be stored at ambient conditions for extended periods of time, or applied to situations where physical stress could be an issue such as air-filters and arid soil conditions.

o Charcoals produced from woods with small macro-pores (diameter of pores between 10 and 15$\mu$m) provided excellent survival conditions for bacteria colonising the pores of these materials.

○ Charcoals produced from bamboo charcoal that contains large pores (diameter of pores ca. 25$\mu$m) offer excellent survival conditions as long as the material is charred at temperatures greater than 650°C.

o In general, more hygroscopic charcoals and charcoals with smaller pore sizes provided better survival conditions for bacteria such as *P. fluorescens.*

**Experiment 5:**

**Survival of bacteria in charcoal at a range of temperatures and relative humidities.**

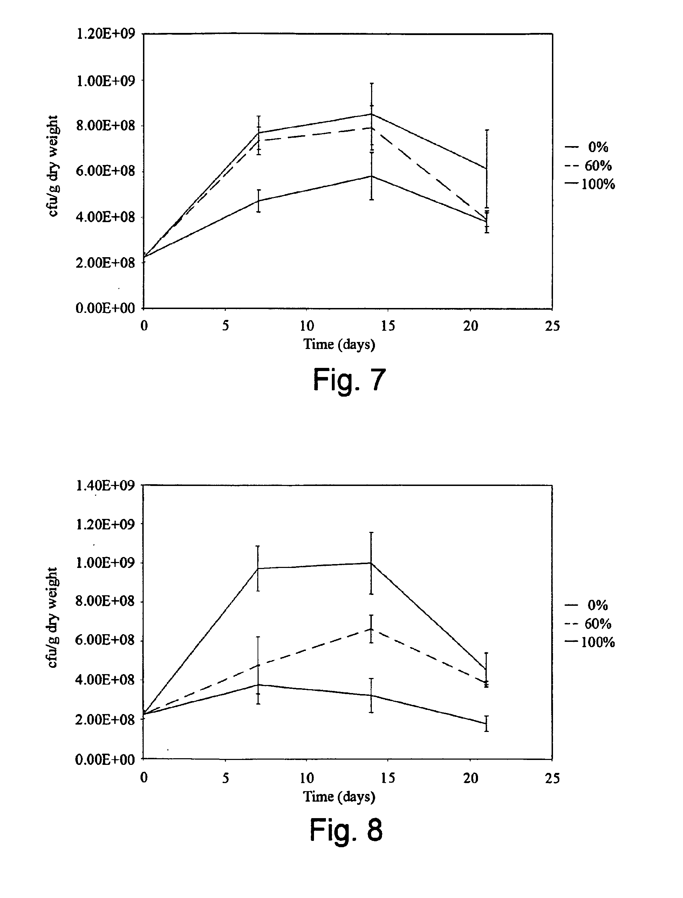

Survival of bacteria in charcoal produced from sweet chestnut at 10°C showed that numbers increased 3 fold at 60 and 100% RH and 2.5 fold at 0% RH during the first 2 weeks. In the following week numbers declined slightly, but were at all relative humidities still twice as high as the initial inoculum. (Fig. 7).

At 20°C bacterial numbers increased more than 3 fold at 100% RH and 2.5 fold at 60% RH. At 0% RH numbers stayed constant. (Fig. 8)

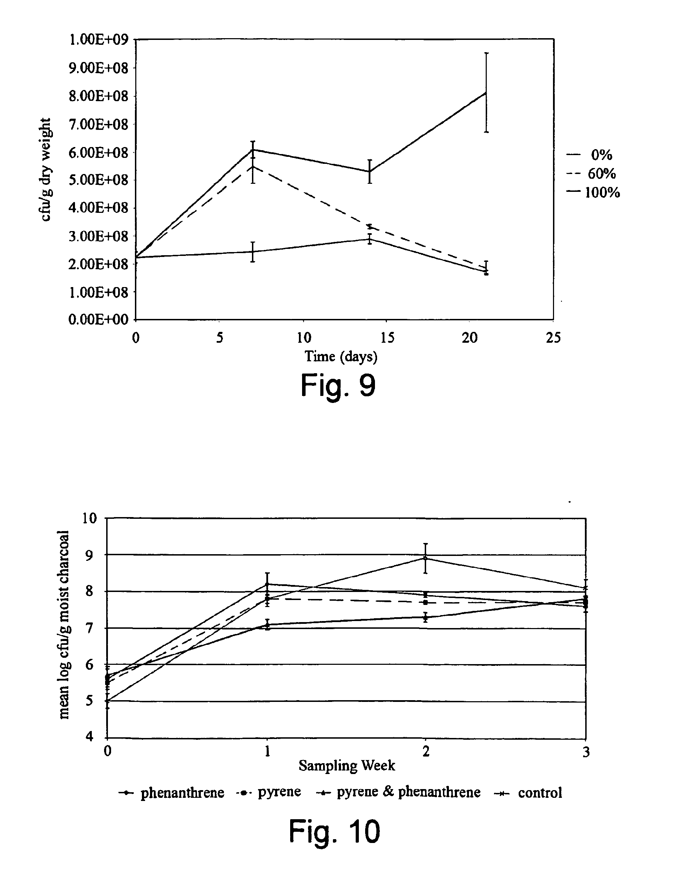

At 30°C bacterial numbers increased 3 fold at 100% RH while numbers at 60% RH initially increase 2 fold but dropped subsequently to the level of the starting population. At 0% RH microbial numbers stayed constant over the duration of the experiment. (Fig. 9)

**Conclusions**

○ Wood charcoal charred at relatively low temperatures (450°C) protects bacteria colonising its internal surfaces from desiccation even at high temperatures (30°C) and relative humidities as low a 0% for at least 3 weeks.

**Example 6**

**Long term survival of bacteria in wood charcoal**

**Introduction**

In order to estimate the survival of bacteria in wood charcoal particles under drying conditions, the following experiment was carried out. Charcoal particles derived from sweet chestnut wood, with a particle size of 1-2 mm were allowed to be colonised by the bacterium *Pseudomonas fluorescens* by soaking the charcoal first in Tryptone Soya Broth (TSB) before inoculation with bacteria. After colonisation of the charcoal, 1g portions (dry weight) of the charcoal were placed in empty Petri dishes (2 replicates) and left for 4 months in the lab (temp 20 C; RH ca 60%). After this time the charcoal in each Petri dish was divided into two portions; one portion was kept dry, and the other was soaked in sterile water for 24 hours, before an estimate of the bacterial population associated with the charcoal was made.

**Results**

**Table 2:** Survival and revival of bacteria *(Pseudomonas fluorescens)* within sweet chestnut charcoal after 4 months storage at 60% RH and a temperature of 20°C

| Treatment | Cfu/g charcoal |
| --- | --- |
| Initial | $5 \times 10^9$ |
| 4 months drying | $1.85 \times 10^7$ |
| 4 months drying + 24h soaking in water | $7.5 \times 10^7$ |

**Conclusion**

o A sizeable population of bacteria can survive within the charcoal for an extended period of time and that once the drying induced stress is removed the population recovers quickly.

**Example 7**

**Creation of specific microbial communities within charcoal**

**Introduction**

To be able to create 'products' that combine the adsorbing properties of charcoal with the ability of microbes to degrade organic pollutants that are adsorbed it is necessary to be able to colonise the charcoal with a large stable community of degraders. Our hypothesis was that effective colonisation of charcoal with specific degraders requires in the charcoal: (a) a source of mineral nutrients, (b) pollutants that are degraded by specific bacteria or groups of bacteria and (c) an inoculum source that contains bacteria of interest. Using the environment within the charcoal as a natural selection criterion will ensure that microbes that are able to establish rapidly into the charcoal will automatically be environmentally competent. Microbes selected into the charcoal can be obtained from a pure culture of previously isolated microbes or can be recruited from an assortment of many bacteria, some of which are able to grow on the organic substrates that are provided with in the charcoal. Whereas it is possible to choose a known hydrocarbon degrader or known degraders of other pollutants to colonise the charcoal, the charging of the charcoal with both BSM and hydrocarbons of choice creates a habitat that will select for those organisms that are capable of degrading these compounds or make them more available.

This is done by adding to the charged charcoal a suspension of bacteria isolated from healthy soil with a large and diverse microbial community. To select a community of pollutant degraders in the charged charcoal a 10% soil suspension in water is made and this is shaken vigorously to separate the microbes from the soil particles; letting the suspension settle for 1 hour; and pouring off the supernatant containing the suspended microbes. This suspension of bacteria is poured over the charged charcoal and the thus inoculated charcoal is incubated for 1 to 4 weeks, depending on the growth rate of the bacteria involved and the complexity of the bio-film, allowing those organisms that can multiply within the charged charcoal particles to form an integrated community of pollutant degraders. In another embodiment the above process if performed using a soil that has been conditioned with pollutants and nutrients to encourage a large community of pollutant degraders to be present before a suspension is created that can be added to charcoal that is previously charged with minerals and vitamins.

Once some of the required microbes enter the charcoal, the nutrients and organic substrates will allow these microbes to multiply. The exact identity of such a community is difficult to assess as there will be a mixture of different species, some of which might be non-culturable under lab conditions, while the identity of others can only be approximated using current species libraries.

To test this hypothesis a range of experiments was set up using charcoal that contained beside a mix of minerals and vitamins, pure hydrocarbons (pyrene and phenanthrene), or a complex mix of hundreds of different hydrocarbons as present in diesel oil. To test the ability of thus impregnated charcoals to enrich for specific microbial communities the following experiments were carried out:

**Establishment of a hydrocarbon degrading population within charcoal using pure cultures**

**Methodology**

In order to study the inoculation of charcoal with PAH (polycyclic aromatic hydrocarbon) degraders, sweet chestnut charcoal was impregnated with minerals as described above, and the PAHs pyrene and phenanthrene. The charcoal was then left to dry before being inoculated with a pure culture of pyrene and phenanthrene degrading bacteria previously isolated from soil. The inoculated charcoal was kept moist with distilled water and incubated at 25 °C. Charcoal samples were then taken over a period of two weeks and the number of pyrene and phenanthrene degrading bacteria on the charcoal were determined.

Two treatments were used:

Treatment 1 - sweet chestnut charcoal was impregnated first with minerals and then with either pyrene or phenanthrene.

Treatment 2 - sweet chestnut charcoal was impregnated first with pyrene or phenanthrene and then with minerals.

The charcoal left over from experiment 1A was then used for another experiment. More minerals were added to the charcoal in order to see if this would increase the numbers of pyrene and phenanthrene degrading bacteria (i.e. were the minerals limiting growth). Minerals were added to the charcoal and then the charcoal was incubated at 25°C. Samples were taken at day 0 (just after adding the minerals), day 1 and day 2 and numbers of pyrene and phenanthrene degrading bacteria were determined.

**Results**

**Table 3:** Colonisation of charcoal particles by a pure population of phenanthrene degraders. Charcoal particles were made of sweet chestnut wood (size .5 - 2 mm) charred at 450°C and impregnated first with minerals before being spiked with phenanthrene (20g kg$^{-1}$ charcoal)

**Table 3**

| PHENANTHRENE Treatment 1 | | | | | |
|---|---|---|---|---|---|
| | Charcoal log cfu/g wet weight | | | | |
| Sampling Day | A | B | C | mean log cfu/g wet weight | Standard Error |
| 0 | 6.64 | 6.45 | 5.78 | **6.29** | **0.26** |
| 1 | 6.95 | 6.95 | 6.81 | **6.90** | **0.05** |
| 2 | 7.95 | 7.72 | 7.90 | **7.86** | **0.07** |
| 7 | 8.36 | 7.93 | 8.59 | **8.29** | **0.19** |
| 14 | 8.30 | 8.26 | 8.70 | **8.42** | **0.14** |

**Table 4:** Colonisation of charcoal particles by a pure population of phenanthrene degraders. Charcoal particles were made of sweet chestnut wood (size .5 - 2 mm) charred at 450°C and impregnated first with with phenanthrene (20g kg$^{-1}$ charcoal) and subsequently with BSM.

**Table 4**

| PHENANTHRENE Treatment 2 | | | | | |
|---|---|---|---|---|---|
| | Charcoal log cfu/g wet weight | | | | |
| Sampling Day | A | B | C | mean log cfu/g wet weight | Standard Error |
| **0** | 6.95 | 6.95 | 6.38 | **6.76** | **0.19** |
| **1** | 6.95 | 6.56 | 6.60 | **6.70** | **0.13** |
| **2** | 8.00 | 7.95 | 7.67 | **7.88** | **0.10** |

(continued)

| PHENANTHRENE Treatment 2 | | | | |
|---|---|---|---|---|
| | Charcoal log cfu/g wet weight | | | |
| Sampling Day | A | B | C | mean log cfu/g wet weight | Standard Error |
| 7 | 7.87 | 8.51 | 7.71 | 8.03 | 0.24 |
| 14 | 7.90 | 7.72 | 7.95 | 7.66 | 0.07 |

**Table 5:** Colonisation of charcoal particles by a pure population of pyrene degraders. Charcoal particles were made of sweet chestnut wood (size .5 - 2 mm) charred at 450°C and impregnated first with minerals before being spiked with pyrene (20g kg$^{-1}$ charcoal)

**Table 5**

| PYRENE Treatment 1 | | | | |
|---|---|---|---|---|
| | Charcoal log cfu/g wet weight | | | |
| Sampling Day | A | B | C | mean log cfu/g wet weight | Standard Error |
| 0 | 5.20 | 5.23 | 5.76 | 5.40 | 0.18 |
| 1 | 6.60 | 5.78 | 6.08 | 6.15 | 0.24 |
| 2 | 7.90 | 6.90 | 6.88 | 7.23 | 0.34 |
| 7 | 8.52 | 7.15 | 7.30 | 7.66 | 0.43 |
| 14 | 8.78 | 6.60 | 7.30 | 7.56 | 0.64 |

**Table 6:** Colonisation of charcoal particles by a pure population of pyrene degraders. Charcoal particles were made of sweet chestnut wood (size .5 - 2 mm) charred at 450°C and impregnated first with with pyrene (20g kg$^{-1}$ charcoal) and subsequently with BSM.

**Table 6**

| PYRENE Treatment 2 | | | | |
|---|---|---|---|---|
| | Charcoal log cfu/g wet weight | | | |
| Sampling Day | A | B | C | mean log cfu/g wet weight | Standard Error 1 |
| 0 | 5.30 | 4.90 | 6.60 | 5.60 | 0.51 |
| 1 | 6.60 | 5.08 | 6.95 | 6.21 | 0.58 |
| 2 | 6.88 | 5.15 | 7.60 | 6.54 | 0.73 |
| 7 | 7.00 | 7.18 | 7.48 | 7.22 | 0.14 |
| 14 | 6.08 | 5.48 | 7.32 | 6.29 | 0.54 |

**Table 7.** Increase of population size of a pure population of phenanthrene degraders by adding double the amount of BSM. One quantity was added before being spiked with phenanthrene (20g kg$^{-1}$ charcoal), while the second quantity was added a week after bacterial inoculation. The data presented is the population size measured after the second addition of BSM.

**Table 7**

| | PHENANTHRENE DEGRADERS log cfu/g moist charcoal | | | | |
|---|---|---|---|---|---|
| **Sampling Day** | Charcoal A | Charcoal B | Charcoal C | **Mean** | **Standard Error** |
| **0** | 7.98 | 8.08 | 8.78 | **8.28** | **0.25** |
| **1** | 8.36 | 8.48 | 7.56 | **8.13** | **0.29** |
| **2** | 8.75 | 8.74 | 9.95 | **9.15** | **0.40** |

Impregnation with minerals first provided better conditions for microbial colonisation compared with adding the BSM after addition of phenanthrene, suggesting that minerals were growth limiting in the case of phenenanthrene (Tables 3,4). Addition of extra minerals resulted in a 10 fold increase of the phenanthrene degrading population to $5 \times 10^9$ bacteria per g charcoal (dry weight). (Table 7)

Impregnation with minerals first had no significant effect on microbial colonisation compared with adding the BSM after addition of pyrene, suggesting that the amount of bio-available pyrene was growth rate limiting and not the amount of minerals (Tables 5,6). More phenanthrene than pyrene degraders could become established on charcoal pieces impregnated with BSM and a similar amount of PAH per g charcoal indicating again that pyrene was less bio-available in the charcoal than pyrene (Tables 2,3,4,5)

**Conclusions**

o A large population of specific hydrocarbon degraders can be established in charcoal using a pure culture of bacteria inoculated onto charcoal that is 'charged' with BSM and a specific hydrocarbon substrate.
o The size of the microbial population within charcoal is dependant on the quantity and 'bio-availability' of the added carbon source and the quantity of available minerals.

**Establishment of a PAH degrading community of bacteria in charcoal using healthy garden soil.**

**Method overview**

Sweet chestnut charcoal charred at 450 °C (particle size ca 2mm) was impregnated with minerals and the poly-aromatic-hydrocarbons (PAHs) pyrene and phenanthrene. The results from previous experiment suggested it was better to add the minerals to the charcoal first, and then add the PAHs (dissolved in acetone). The charcoal was dried before being inoculated with a suspension of bacteria extracted from soil. The charcoal was kept in a 250ml glass conical flask, covered with a paper cup and incubated at 25°C. The inoculated charcoal was kept moist by adding 5-10ml of distilled water to maintain microbial activity.

**Treatments**

• **20,000ppm phenanthrene** on mineral impregnated charcoal.
• **20,000ppm pyrene** on mineral impregnated charcoal.
• **10,000ppm phenanthrene** + **10,000ppm pyrene** on mineral impregnated charcoal.
• **Control** mineral impregnated charcoal without PAHs.

The following operating procedures were used to obtain the above treatments

**1. Impregnating the Charcoal -minerals added first, PAHs added last**

1. Weigh out 4 x 32g portions of sweet chestnut charcoal >0.5mm, <2mm diameter, and place in 250ml glass conical flasks.
2. Add 150ml of sterile mineral solution (basal salts medium without agar) to each 32g of charcoal in the 250ml flask and shake at 100rpm for 2 days.

3. Filter the charcoal and leave to dry overnight at room temperature.

4. Once dry, separate into 8g portions and place each in 250ml conical flasks.

5. Dissolve the following amounts of the PAHs (pyrene or phenanthrene) in acetone and add to the 8g portion of charcoal.

**Table 8**

| PAH | Concentration (ppm) | mg to dissolve | acetone (ml) |
|---|---|---|---|
| phenanthrene | 20,000 | 160 | 20 |
| pyrene | 20,000 | 160 | 20 |
| phenanthrene & pyrene | 10,000 | 80 | 20 |
| | 10,000 | 80 | |

6. Seal the flask with a rubber bung and shake the flask for 5 minutes by hand. Leave to evaporate in the fume cupboard.

## 2. Inoculating the Charcoal with Bacteria from Soil

7. Collect 100g of non-contaminated healthy top soil. (soil from around the bases of trees were taken (same site as experiment 2).

8. Use a coarse sieve to remove stones, twigs, leaves, worms, etc.

9. Make up 16 x 1:10 soil suspensions of 2g soil (wet weight) in 20ml sterile quarter strength Ringer's solution.

10. Leave the soil to settle out.

11. Pour the supernatant (containing bacteria) into a centrifuge tube and centrifuge at 3,000 rpm for 10 minutes.

12. Discard the supernatant.

13. Re-suspend each pellet (containing bacteria) in 20ml sterile quarter strength Ringer's solution.

14. Add the bacterial suspension to the 8g portion of charcoal and mix.

15. Cover flask with a paper cup and incubate at 25°C until needed for sampling.

16. Check the charcoal every few days to ensure that it remains moist. At signs of drying add 5-10ml of distilled water to maintain microbial activity. Incubate for 3 weeks to select degraders onto the charcoal

## 3. Sampling

17. Take charcoal samples to determine the total number of bacteria and the number of PAH degrading bacteria (using spray plates) on the charcoal at weeks 0, 1, 2 and 3.

18. Determine the concentration of PAHs on the charcoal at week 3 using Soxhlet extraction and GC-MS.

## 4. Determine total no. of bacteria-weeks 0, 1, 2 & 3

19. Collect flasks containing the inoculated charcoal from the 25°C incubator.

20. From each flask / sampling time weigh out 1g of inoculated charcoal.

21. Place 10ml of sterile quarter strength Ringer's solution into a mortar. Add the 1g of charcoal and using the pestle and mortar grind the charcoal in the solution to release the bacteria.

22. Using a funnel pour the charcoal suspension from the mortar into a universal bottle.

23. Prepare a serial dilution series..

24. Take 0.1ml from the appropriate dilutions and spread onto 1% TSA plates. Use 100% ethanol and a Bunsen burner flame to sterilise a glass rod to spread the plates.

25. Incubate the TSA plates at 25°C for 10 days and make a total count.

5.Quantification of PAH Degrading Bacteria (Spray Plates) - weeks 0, 1, 2 & 3

26. Use the diluents that were made for the total bacterial counts.

27. Take 0.1ml from the appropriate dilutions and spread onto basal salts plates. Use 100% ethanol and a Bunsen burner flame to sterilise a glass rod to spread the plates.

28. Leave the plates to dry overnight at 25°C.

29. In a fume cupboard; dissolve 0.5g of pyrene or phenanthrene (depending on the degrader to be enumerated) in 250ml of diethyl-ether.

30. Cover the spread plates and incubate at 25°C. Phenanthrene covered plates should be left for 14 days, being counted every 2-3 days, with a final count on the 14th day. Pyrene covered plated are left for 21 days, being counted every 2-3 Jays, with a final count on the 21st day.

**Results**

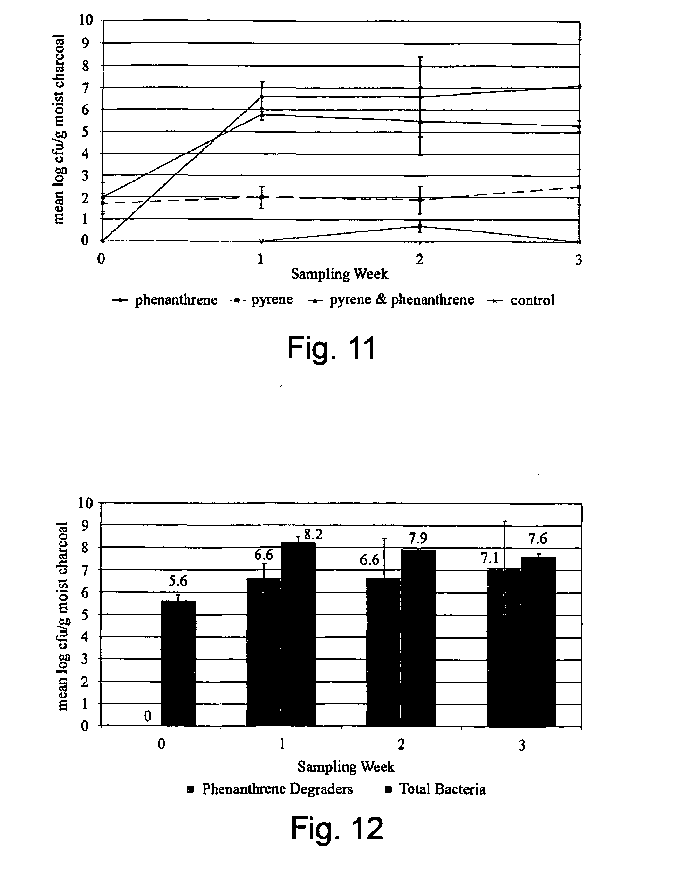

See Figures 10-13.

**Conclusions**

o Addition of both minerals and a specific (hydro)carbon source to charcoal results in the establishment of a community of microorganisms capable of utilising that carbon source when a mixed suspension of bacteria isolated from soil is added to the thus impregnated charcoal.

o Using phenanthrene impregnated charcoal as an example, within 3 weeks more than 30% of the bacteria that colonised the thus impregnated charcoal consisted of phenanthrene degraders - when no selective carbon substrate was added none of the bacteria colonising charcoal could be classified as phenanthrene degraders.

**Example 8** (this example is not in accordance with the claimed invention)

**Establishment of a Large Community of Diesel Degrading Bacteria in Charcoal**

**Introduction**

The aim of this experiment was to establish a large community of diesel degrading bacteria in charcoal. A concentrated mineral solution was added to sweet chestnut charcoal (Table 1). It was hypothesised that natural healthy soil contains a large variety of micro-organisms, including ones that are able to degrade hydrocarbons present in diesel oil, being it in low numbers. To increase the number of organisms that are capable of degrading diesel hydrocarbons in healthy soil, this soil can be amended with low levels of diesel and manure. It is therefore expected that a microbial in inoculum obtained from the latter soil would have more hydrocarbon degraders than non-spiked soil and if brought into contact with charcoal that was spiked with diesel and minerals these organisms would rapidly establish inside the charcoal, producing a bioreactor that can subsequently be used to degrade diesel spills. Furthermore, it was hypothesised that once a suitable diesel degrading community of diesel degraders had established on the charcoal this community could be used to inoculate 'clean' charcoal to obtain effective bioreactors.

**Method**

Sweet Chestnut wood was charred in a furnace at 450°C. The resulting charcoal was then broken up and graded to produce charcoal particles of 0.5 - 2mm diameter. 20g portions of charcoal were placed in conical flasks and 20ml of concentrated mineral solution was added to each portion of charcoal (Table 1) allowing charcoal to become charged with minerals while leaving enough open space for the microbial inoculum suspended in water.

The charcoal was then inoculated with bacteria using the following method: 3g of the soil was suspended in 10ml sterile quarter strength Ringers solution, the suspension was shaken to dislodge the microbes from the soil particles and the soil particles were allowed to settle for 10 minutes. The supernatant of this suspension was then added to charcoal charged with minerals. This would have resulted in the charcoal being inoculated with a variety of microbes, most of which would be incapable of diesel degradation.

To encourage diesel degrading bacteria to multiply within the inoculated charcoal, 1g of diesel in 10ml distilled water was shaken and poured over the 20g of inoculated charcoal.

Three treatments and one control were used:

**Control (no inoculum)** = charcoal + 10ml sterile quarter strength Ringer's solution + minerals + diesel.

**Treatment 1 (soil)** = charcoal + minerals + soil extract + diesel.

Method: 1kg of silty loam soil that had been amended with compost was collected from the university campus, the soil was sieved and incubated for 2 days at 20°C. 3g of the soil was suspended in 10ml sterile quarter strength Ringers solution, the solution was mixed and the soil particles were allowed to settle for 10 minutes before the supernatant was then added to 20g of charcoal.

**Treatment 2 (soil, 1% (w/w) chicken pellets, 5% (w/w) diesel)** = charcoal + minerals + soil extract from soil amended with diesel & chicken manure.

Method: 1kg soil was collected from the university campus, the soil was sieved and 50g (1% w/w) of chicken manure and 50g (5% w/w) of diesel oil was mixed into the soil. The soil was incubated for 2 days at 20°C. 3g of the soil was suspended in 10ml sterile quarter strength Ringers solution, the solution was mixed and the soil particles were allowed to settle. The supernatant was then added to the 20g of charcoal.

**Treatment 3 (recycled charcoal)** = charcoal + minerals + solution of bacteria from charcoal from a previous experiment + diesel.

Method: Charcoal with a large diesel degrading community was obtained using the method described in treatment 2. Three g of this charcoal was ground in 10ml of sterile quarter strength Ringer's solution. This suspension was then poured onto 20g charcoal.

To estimate diesel degradation the flasks with charcoal were incubated at 25°C. The amount of carbon dioxide produced by each 20g portion of charcoal was then measured for 2 weeks. When the charcoal began to dry 10ml of dilute mineral solution was added.

## Results & Conclusions

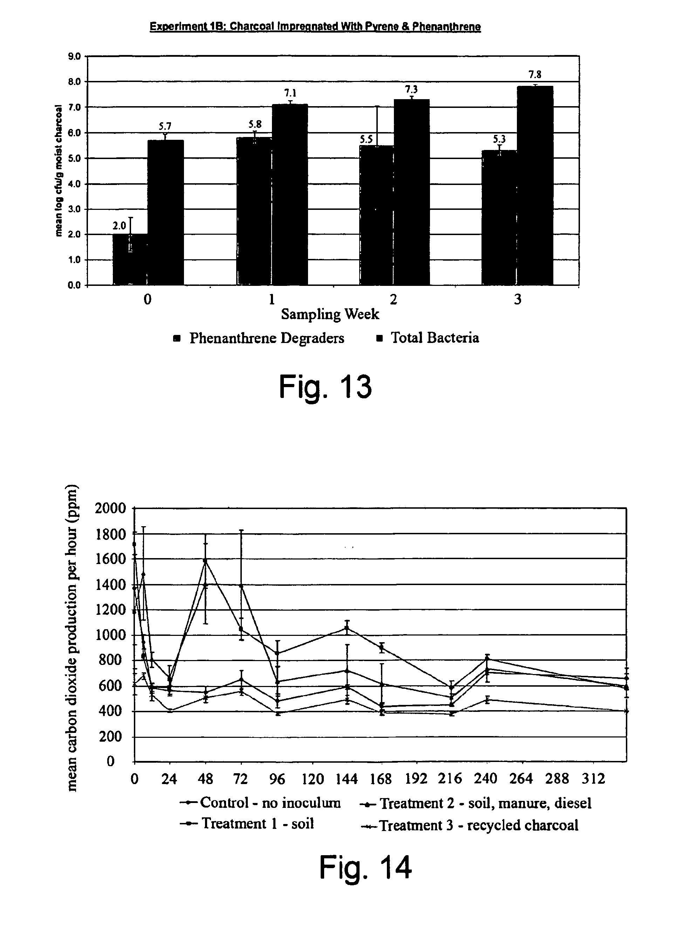

With reference to Figure 14, charcoal that had been inoculated with a soil solution (treatments I & 2) produced significantly more carbon dioxide per hour than the control. This suggests that bacteria from soil had colonised the charcoal and were degrading the diesel. Carbon dioxide production from the inoculated charcoal peaked after 48 - 72 hours (2 - 3 days) and then declined so that by 2 weeks the inoculated charcoal was producing the same amount of carbon dioxide per hour as the control. The graph suggests that most of the diesel had been degraded by day 4 (96 hours), with complete diesel degradation by day 9 (216 hours). Especially the treatment where the charcoal had been inoculated with bacteria obtained from soil spiked with diesel and chicken manure seem to work particularly well with almost all diesel being degraded after 4 days. In treatment 3 (recycled charcoal) the amount of carbon dioxide production per hour was similar to that of the control, which implies that bacteria isolated from previously inoculated charcoal provides a poor inoculum for the next batch of charcoal bio-reactors. The reason for this poor performance is not clear, but it is possible that the bacterial biofilm that is formed on charcoal is difficult to dislodge, making it difficult to obtain a new inoculum that can be used for further inoculations of charcoal.

## Conclusions

o Both natural soil and soil that was enriched for diesel degraders were effective for the establishment of a large hydrocarbon degrading community within the charcoal

**Example 9**

**Efficacy of Charcoal Bioreactors for the decontamination of Diesel Contaminated Sand**

**Introduction**

This experiment was set up to test the efficacy of charcoal bioreactors to decontaminate sand that was contaminated with 5% (w/w) diesel oil. Application rates were 5 and 10% by soil volume and bioreactor treatments were compared with amendments of non-inoculated charcoal as well as with a non-amended control treatment.

**Methodology**

Creating Charcoal Bioreactors (inoculated charcoal)

1. Collect 1kg of healthy garden soil, sieve and place in an aerated container.
2. Add 10g chicken pellets ground into 40ml of water.
3. Add 50g of diesel to the soil and incubate at 20°C for 7 days.
4. To make 60g (~396ml) of inoculated charcoal; weigh out 3 x 20g of sweet chestnut charcoal (particle size between 0.5 and 2mm) into a 250ml conical flask.
5. Add 20ml of concentrated mineral solution to each 20g of charcoal seal the flask with a bung and shake by hand.
6. Make 3 soil suspensions of 3g soil in 10ml sterile quarter strength Ringers solution. Leave the soil particles to settle, then pipette out the supernatant (containing bacteria) and add to the charcoal.
7. To each 20g of charcoal add 1g of diesel dissolved in 10ml distilled water; mix the diesel and water well, and then pour onto the charcoal. Shake the flask.
S. Incubate at 25°C and measure carbon dioxide production after 2 and 4 days
9. Just before the charcoal is added to the sand in the propagators, add 10ml of concentrated mineral solution to each 20g portion of charcoal.

**Preparing the Sand**

1. Weigh out 15.6kg of builders sand (Rugby Building Sand; B&Q).
2. Add 669ml concentrated liquid fertiliser to the 15.6kg of sand.
3. Separate the sand into two 7.8 kg portions (one for 0% diesel contamination and one for 5% diesel contamination).
4. For the 5% diesel (w/w): add 390g diesel to 7.8kg of sand and mix well.
5. Separate the 2 sands (0% diesel and 5% diesel) into 65g portions and put into labelled propagator trays (65g sand per section of tray).

**Adding the Charcoal**

1. There are 2 types of charcoal; inoculated (bioreactors) and non-inoculated (charcoal).
2. For 5% (v/v): add 2.75ml of charcoal to 65g (55ml) sand, for each section of the propagator. Mix the charcoal into the sand.
3. For 10% (v/v): add 5.5ml of charcoal to 65g (55ml) sand, for each section of the propagator. Mix the charcoal into the sand.

**Results**

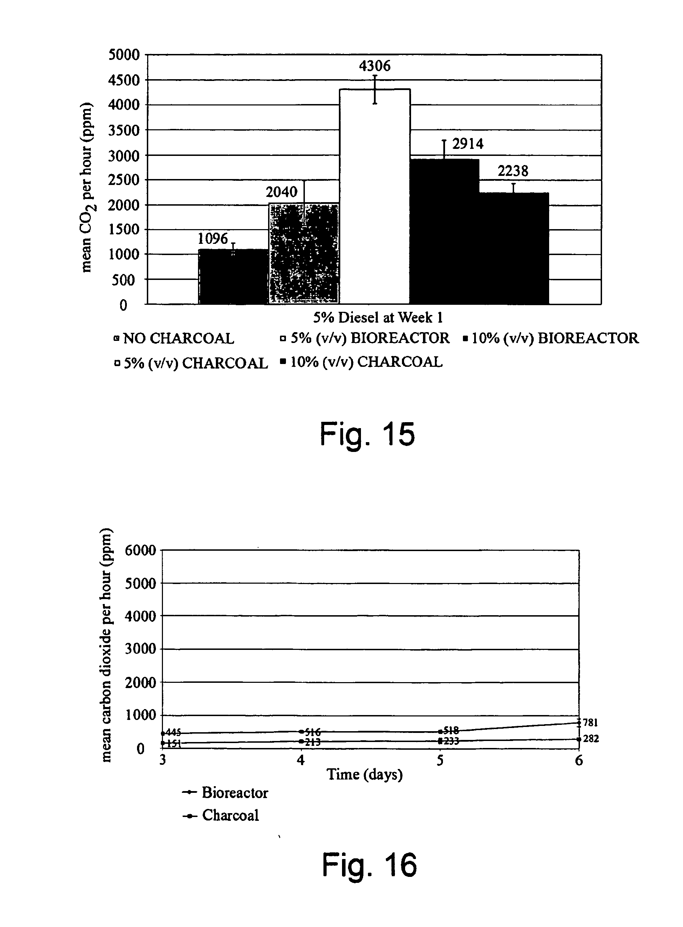

See Figure 15.

Conclusions

o Charcoal addition to diesel contaminated soil led to a significant increase in the rate of diesel degradation.

o Addition of 5% (v/v) charcoal bioreactors to diesel contaminated sand led to a doubling of the rate of diesel degradation compared to sand that was amended with just charcoal and a 4 fold increase of diesel degradation compared to non-amended sand.

o Addition of 10% charcoal, inoculated or not, led to a 2-3 fold increase in the rate of diesel degradation, but inoculation did not enhance the rate of diesel Degradation. The reason for this lies in the fact that the sand systems easily become anaerobic preventing microbial oxidation of the hydrocarbons.

**Example 10**

**Efficacy of Charcoal Bioreactors to Remediate Diesel Spills**

**Introduction**

Currently small oil spills as occur at garage forecourts are often dealt with by covering the spill with sand. Not only is sand a poor adsorbing material, but it is unlikely to contain large numbers of hydrocarbon degraders. One of the applications where charcoal bio-reactors could be used is for the adsorption and subsequent degradation of a variety of spills in environments that are low in naturally occurring hydrocarbon degraders. To test (a) how much hydrocarbon (diesel) can be adsorbed by charcoal and (b) how quickly this can be degraded subsequently. Because hydrocarbon degradation is in the main via aerobic oxidation of the various hydrocarbons it was expected that adsorption of large quantities of diesel onto charcoal would lead to reduced rates of degradation. Furthermore, it was expected that oxidation of large quantities of hydrocarbons required extra minerals.

**Methodology**

**Determining M.A.D.**

1. Weigh out 3 x 10g of Sweet Chestnut charcoal (<2mm, >0.5mm diameter) into 500ml conical flasks.
2. Add 100ml of diesel to each flask and seal with foil.
3. Leave the charcoal to soak for overnight in the fume hood.
4. Place the charcoal on a sieve and allow the diesel to drain off - catch the diesel. Use sieves from the soil lab - fine sieve to so that small charcoal particles don't fall into the diesel. Leave to drain for 2 hours.
5. Once drained, weigh the charcoal to determine how much diesel has been absorbed.

**M.A.D. Results**

Weight of charcoal soaked in diesel:

Replicate 1 = 48.5g - 10g charcoal = 38.5g
Replicate 2 = 46.7g - 10g charcoal = 36.7g
Replicate 3 = 48.3g - 10g charcoal = 38.3g

Mean weight of diesel absorbed by charcoal:

$$(38.5 + 36.7 + 38.3) / 3 = 37.83g = 38g$$

The maximum amount of diesel (M.A.D.) that can be adsorbed onto charcoal was found to be 38g diesel per 10g charcoal.

**Charcoal Bioreactor Efficacy**

Treatments and replications:

1. Diesel concentrations (No diesel, 0.1 MAD, 0.2 MAD, 0.3 MAD, 0.4 MAD, 0.5 MAD).
2. 2 types of Charcoal: inoculated (bioreactor), un-inoculated.
3. 4 replicates of each of the above
4. Number of flasks: 6 x 2 x 4 = 48

Creation of Charcoal Bioreactors (inoculated charcoal)

1. Collect 1kg of healthy garden soil, sieve the soil and place in an aerated container.
2. Add 10g chicken pellets ground into 40ml of water.
3. Add 50g of diesel to the soil and incubate at 20°C for 14 days.
4. Weigh out 12 x 20g of sweet chestnut charcoal (particle size between 0.5 and 2mm) into 250ml conical flasks.
5. Add 20ml of concentrated mineral solution (Table 1) to each 20g of charcoal; seal the flask with a bung and shake by hand to distribute minerals evenly.
6. Make 12 soil suspensions of 3g soil in 10ml sterile quarter strength Ringers solution. Leave the soil particles to settle, then pipette out the supernatant (containing bacteria) and add to the charcoal.
7. To each 20g of charcoal add 1g of diesel dissolved in 10ml distilled water; mix the diesel and water well, and then pour onto the charcoal. Shake the flask.
8. Incubate at 25°C and measure carbon dioxide production after 2 and 3 days.
9. Just before the charcoal bioreactors are needed, add 10ml of concentrated mineral solution to each 20g portion of charcoal.

Experimental set up

1. Inoculated charcoal (bioreactors); weigh out 24 x 10g of the bioreactors and place in labelled 250ml conical flasks.
2. Un-inoculated charcoal; weigh out 24 x 10g of Sweet Chestnut charcoal (<2mm, >0.5mm diameter) and place in labelled 250ml conical flasks.
3. Add diesel at the following concentrations;

**Table 9**

| MAD* Value | g diesel per 10g charcoal |
|---|---|
| 0 | 0 |
| 0.1 | 3.8 |
| 0.2 | 7.6 |
| 0.3 | 11.4 |
| 0.4 | 15.2 |
| 0.5 | 19 |
| **MAD* = maximum amount of diesel that can be adsorbed onto charcoal** | |

4. Add 7g of slow release fertiliser per flask (10g charcoal per flask).
5. *Slow release fertiliser: Miracle-Gro Osmocote Granules Controlled Release Plant Food, Scotts Company (UK) Limited, Godalming, Surrey.*
6. Leave the flasks in a fume hood overnight to reduce volatile compounds in the flasks.
7. Incubate at 20°C in the dark
8. Quantify respiration

With reference to See Figure 16, two to three times more $CO_2$ evolved from charcoal particles that were colonised by bacteria than from those that were not inoculated. This can be explained by the fact that inoculated particles contained usable carbon in the form of dead microbial biomass and possibly some residual diesel oil. The amount of respiration from inoculated charcoal without any extra added diesel oil can be defined as 'base respiration'.

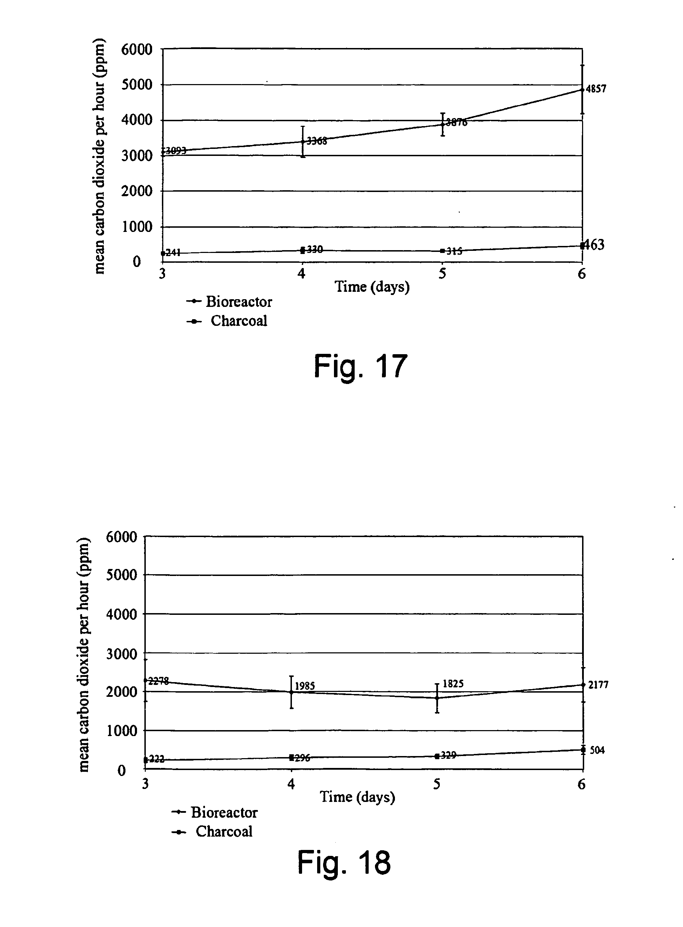

17 times more $CO_2$ evolved from charcoal particles that were colonised by bacteria than from those that were not inoculated (P<0.001) (Fig. 17). Compared with inoculated control charcoal (0 M.A.D.; Fig 16) a 7 fold increase in respiration occurred in charcoal that had been allowed to soak up 3.8 g diesel per 10 g charcoal (0.1 M.A.D). Six times more $CO_2$ evolved from charcoal particles that were colonised by bacteria than from those that were not inoculated (P<0.001) (Fig. 18). Compared with inoculated control charcoal (0 M.A.D.; Fig 16) a 3.7 fold increase in respiration occurred in charcoal that had been allowed to soak up 7.6 g diesel per 10 g charcoal (0.2 M.A.D).

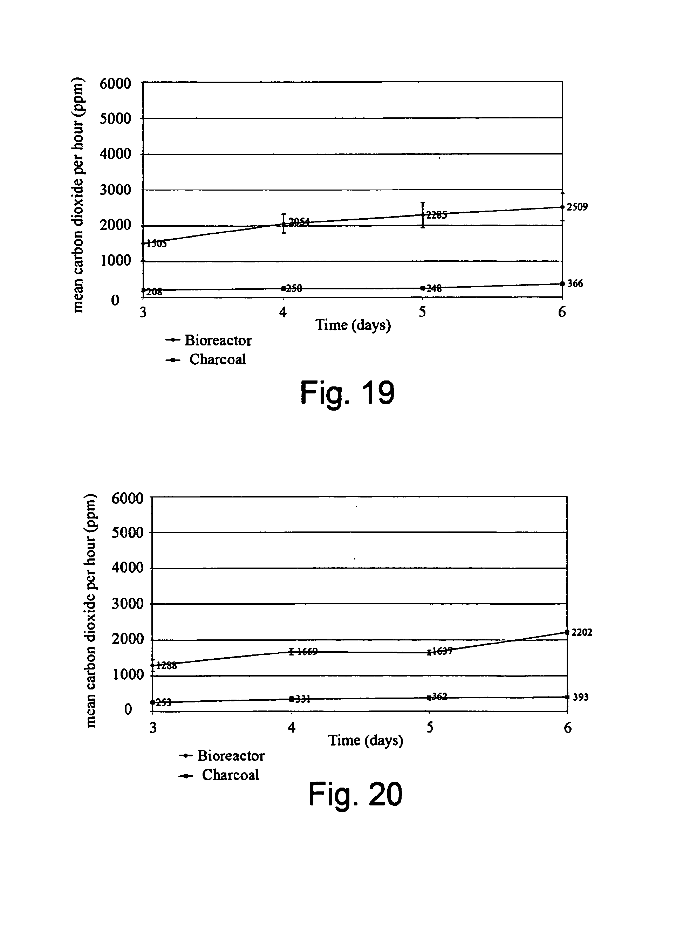

Eight times more $CO_2$ evolved from charcoal particles that were colonised by bacteria than from those that were not inoculated (P<0.001) (Fig. 19). Compared with inoculated control charcoal (0 M.A.D.; Fig 16) a 3.7 fold increase in respiration occurred in charcoal that had been allowed to soak up 11.4 g diesel per 10 g charcoal (0.3 M.A.D).

Five times more $CO_2$ evolved from charcoal particles that were colonised by bacteria than from those that were not inoculated (P<0.001) (Fig. 20). Compared with inoculated control charcoal (0 M.A.D.; Fig 16) a 3 fold increase in respiration occurred in charcoal that had been allowed to soak up 15.2 g diesel per 10 g charcoal (0.4 M.A.D).

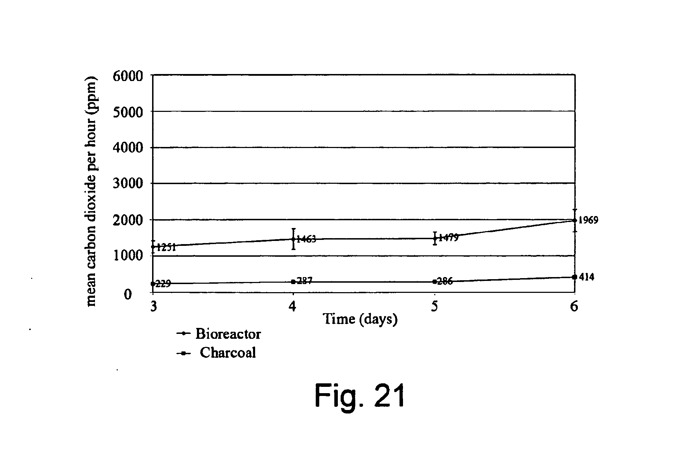

Five times more $CO_2$ evolved from charcoal particles that were colonised by bacteria than from those that were not inoculated (P<0.001) (Fig. 21). Compared with inoculated control charcoal (0 M.A.D.; Fig 16) a 3 fold increase in respiration occurred in charcoal that had been allowed to soak up 19 g diesel per 10 g charcoal (0.5 M.A.D).

Conclusions

o Use of charcoal bioreactors enhanced degradation of diesel oil significantly (P, 0.001), irrespective of the amount of diesel adsorbed
o Absorption of 10% of the maximum amount of diesel onto charcoal bioreactors (0.4 g diesel/g charcoal) led to a more than 10 fold increase in the the degradation rate of diesel oil.
o Larger quantities of diesel adsorption (between 20 and 50% of the maximum adsorbed quantity (between 0.8 and 2 g diesel / g charcoal) led to an increase in the rate of diesel degradation over a 6 day period by more than 300%.

**Claims**

1. A method for treating a site or environment in which at least one selected environmental pollutant is present the method comprising:

   colonising charcoal with microbes, to form a community of different cooperating microbes in a biofilm, wherein the community of microbes is isolated from a naturally occuring environment in the presence of a selective amount of the at least one pollutant, wherein the microbial community is obtained after incubating a preparation of the source charcoal with an amount of the selected pollutant sufficient to preferentially select for tolerant microbes, the microbes being capable of metabolising the at least one pollutant wherein a selective amount of the pollutant is present in the charcoal; and
   introducing the microbially colonised charcoal to the site or environment to be treated such that the colonising microbes are able to metabolise the at least one pollutant from the site or environment to be treated.

2. A method according to claim 12, comprising subsequent addition of: a) further nutrients, and/or b) further colonised material and/or c) further uncolonised material.

3. A method according to claim 11 or 12, wherein the charcoal is removed and 35 further processed by:

   i) heavy metal chelation; and/or
   ii) elevating the temperature of the charcoal to encourage microbial growth; and/or
   iii) dosing with minerals and/or nutrients.

4. A method according to any of claims 1 to 3, for the treatment of; contaminated soils both *in situ* and *ex situ*, water, sewage, sewage sludge, oil waste, oil spills, petroleum spills, landfill, landfill leachate, biodegradeable waste, industrial discharge, and/or airborne contaminants.

5. A method according to any of claims 1 to 4 where the charcoal is colonised with a biological control agent, a plant growth promoting organism, a pro-biotic organism or a nitrogen fixing organism using the charcoal as a carrier of such

**EP 1 968 898 B1**

## Patentansprüche

1. Verfahren zur Behandlung eines Geländes oder einer Umgebung, in dem bzw. der mindestens ein ausgewählter Umweltschadstoff vorliegt, wobei das Verfahren Folgendes umfasst:

   Kolonisieren von Holzkohle mit Mikroben, um eine Gemeinschaft unterschiedlicher zusammenwirkender Mikroben in einem Biofilm zu bilden, wobei die Gemeinschaft von Mikroben aus einer natürlich vorkommenden Umgebung in Gegenwart einer selektiven Menge des mindestens einen Schadstoffs isoliert wird, wobei die Mikrobengemeinschaft nach Inkubieren eines Präparats der Quellholzkohle mit einer Menge des ausgewählten Schadstoffs, die dazu ausreicht, um auf tolerante Mikroben bevorzugt zu selektieren, erhalten wird, wobei die Mikroben den mindestens einen Schadstoff metabolisieren können, wobei eine selektive Menge des Schadstoffs in der Holzkohle vorliegt; und
   Einbringen der mit Mikroben kolonisierten Holzkohle in das zu behandelnde Gelände oder die zu behandelnde Umgebung, so dass die kolonisierenden Mikroben den mindestens einen Schadstoff aus dem zu behandelnden Gelände oder der zu behandelnden Umgebung metabolisieren können.

2. Verfahren nach Anspruch 1, das die anschließende Zugabe von: a) weiteren Nährstoffen und/oder b) weiterem kolonisierten Material und/oder c) weiterem nicht kolonisierten Material umfasst.

3. Verfahren nach Anspruch 1 oder 2, wobei die Holzkohle entfernt wird und durch:

   i) Bilden von Schwermetallchelaten und/oder
   ii) Erhöhen der Temperatur der Holzkohle, um das Mikrobenwachstum zu fördern; und/oder
   iii) Zumessen von Mineralien und/oder Nährstoffen weiter verarbeitet wird.

4. Verfahren nach einem der Ansprüche 1 bis 3 zur Behandlung von kontaminierten Erdböden sowohl in situ als auch ex situ, Wasser, Abwasser, Klärschlamm, Ölabfall, Öllachen, Erdöllachen, einer Deponie, Deponiesickerwasser, biologisch abbaubarem Abfall, Industrieabflüssen und/oder Schwebstoffen.

5. Verfahren nach einem der Ansprüche 1 bis 4, wobei die Holzkohle mit einem biologischen Pflanzenschutzmittel, einem das Pflanzenwachstum fördernden Organismus, einem probiotischen Organismus oder einem stickstoffbindenden Organismus unter Verwendung der Holzkohle als einem Träger derartiger Organismen kolonisiert wird.

## Revendications

1. Procédé pour le traitement d'un site ou d'un environnement dans lequel au moins un polluant environnemental choisi est présent, le procédé consistant à :

   coloniser du charbon de bois par des microbes pour former une colonie de différents microbes coopérant dans un biofilm, dans lequel la colonie de microbes est isolée à partir d'un environnement naturel en présence d'une quantité sélective du ou des polluants, dans lequel la colonie microbienne est obtenue après incubation d'une préparation de la source de charbon de bois avec une quantité du polluant choisi, suffisante pour sélectionner préférentiellement des microbes tolérants, lesdits microbes étant capables de métaboliser le ou les polluants, dans lequel une quantité sélective du polluant est présente dans le charbon de bois ; et
   introduire le charbon de bois colonisé par des microbes dans le site ou l'environnement à traiter de telle façon que les colonies de microbes soient capables de métaboliser le ou les polluants du site ou de l'environnement à traiter.

2. Procédé selon la revendication 1, comprenant l'ajout subséquent :

   a) d'éléments nutritifs supplémentaires, et/ou b) de matériau colonisé supplémentaire et/ou c) de matériau non colonisé supplémentaire.

3. Procédé selon la revendication 1 ou 2, dans lequel le charbon de bois est éliminé et de nouveau traité par :

   i) chélation de métaux lourds ; et/ou
   ii) élévation de la température du charbon de bois pour favoriser la croissance microbienne ; et/ou

iii) dosage avec des minéraux et/ou des éléments nutritifs.

4. Procédé selon l'une quelconque des revendications 1 à 3, pour le traitement de sols contaminés in situ et ex situ, d'eau, d'eaux usées, de boue d'eaux usées, de déchets huileux, de déversements d'huile, de déversements de pétrole, de sites d'enfouissement, de lixiviat de sites d'enfouissement, de déchets biodégradables, de rejets industriels, et/ou de contaminants aéroportés.

5. Procédé selon l'une quelconque des revendications 1 à 4, dans lequel le charbon de bois est colonisé par un agent de régulation biologique, un organisme favorisant la croissance végétale, un organisme probiotique ou un organisme fixateur d'azote en se servant du charbon de bois en tant que support de tels organismes.

Fig. 1

Fig. 2

**Fig. 3**

**Fig. 4**

Fig. 5

Fig. 6

Fig. 7

Fig. 8

Fig. 9

-•- phenanthrene  -•- pyrene  -•- pyrene & phenanthrene  -•- control

Fig. 10

Fig. 11

Fig. 12

**Experiment 1B: Charcoal Impregnated With Pyrene & Phenanthrene**

■ Phenanthrene Degraders    ■ Total Bacteria

## Fig. 13

→ Control - no inoculum    → Treatment 2 - soil, manure, diesel
→ Treatment 1 - soil    → Treatment 3 - recycled charcoal

## Fig. 14

Fig. 15

Fig. 16

Fig. 17

Fig. 18

Fig. 19

Fig. 20

Fig. 21

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6451580 A, Takagi
- US 6451580 B
- JP 2005021748 B
- JP 8001133 B
- JP 8252086 B
- DE 19928087
- JP 11104600 B
- JP 8309375 B
- US 5918333 A

**Non-patent literature cited in the description**

- **VOUDRIAS EA.** *Global Nest: the Int. J.,* 2001, vol. 3 (1), 1-10